# EUROPEAN PATENT APPLICATION

(11) **EP 2 361 989 A1**
(43) Date of publication of application: **31.08.2011**
(21) Application number: 10002022.1
(22) Date of filing: 26.02.2010
(51) Int. Cl.: C12Q 1/68

(54) **Assays and kits for serotyping Pseudomonas aeruginosa and oligonucleotide sequences useful in such methods and kits**

(71) Applicant: Kenta Biotech AG, 3018 Bern (CH)
(72) Inventor: Emrich, Thomas, Dr., 82393 Iffeldorf (DE); Rudolf, Michael, Dr., 3063 Ittigen (CH); Koch, Holger, Dr., 8005 Zürich (CH)
(74) Representative: Grünecker, Kinkeldey, Stockmair & Schwanhäusser Anwaltssozietät

(57) **Abstract**

The present invention relates to assays, kits and oligonucleotides for the detection of *Pseudomonas aeruginosa* for a fast, sensitive and reliable detection of *Pseudomonas aeruginosa* in a species- and serotype-specific manner. In particular, the present invention provides an assay for the serotype-specific detection of *Pseudomonas aeruginosa,* a kit for the serotype-specific detection of *Pseudomonas aeruginosa,* as well as oligonucleotides useful in such assay or kit. The present invention further relates to the use of *Pseudomonas aeruginosa* serotype specific antibodies for serotype specific treatment of *Pseudomonas aeruginosa* infection in a patient detected for said specific *Pseudomonas aeruginosa* serotype with such an assay or kit.

## Description

### Field of the Invention

The present invention relates to an assay for the serotype-specific detection of *Pseudomonas aeruginosa,* a kit for the serotype-specific detection of *Pseudomonas aeruginosa,* as well as oligonucleotides useful in such assay or kit. The present invention further relates to the use of *Pseudomonas aeruginosa* serotype specific antibodies for serotype specific treatment of *Pseudomonas aeruginosa* infection in a patient suffering from a disease caused by said particular *Pseudomonas aeruginosa* serotype.

### Background of the Invention

*Pseudomonas aeruginosa* is a ubiquitous gram-negative environmental bacterium found in fresh water and soil. It is a classical opportunistic pathogen that does not normally pose a threat to the immunocompetent host, who clears it by means of opsonising antibodies and phagocytosis. However, cystic fibrosis patients and immunocompromised individuals - including burn victims, intubated patients in intensive care units, cancer and AIDS patients, as well as patients undergoing organ transplantation - are at particularly high risk of contracting nosocomial infections such as a *Pseudomonas aeruginosa* infection. Acute infections in these patients can rapidly have fatal courses, as shown in the attributable mortality rate (33-50%) of ventilator-associated pneumonia (VAP) caused by *Pseudomonas aeruginosa.* Even more importantly, the resistance of *Pseudomonas aeruginosa* against antibiotics is increasing significantly. The proportion of *Pseudomonas aeruginosa* isolates resistant to ceftazidime, a third generation cephalosporin, has for example increased from 12% in 1995 to 29% in 2001. Together with methicillin-resistant S. *aureus* (MRSA) and vancomycin-resistant *enterococci* (VRE), *Pseudomonas aeruginosa* is responsible for up to 34% of all nosocomial infections, which have increased from 7.2/1000 patient days in 1975 to 9.8/1000 patient days in 1995. Among the most frequently observed forms of nosocomial infection are blood-stream infections and pneumonia.

In the context of increasing antibiotic resistance, the use of human monoclonal antibodies for passive immunotherapy against *Pseudomonas aeruginosa* infections is a new promising approach. It has been shown that antibodies targeting the O-antigen specific carbohydrates - the main component on the surface of gram-negative bacteria - are important mediators in specific protection against infections caused by *Pseudomonas aeruginosa.* As the outer carbohydrate layer (O-antigen) differs from serotype to serotype, several serotype-specific antibodies are needed to provide broad protection against *Pseudomonas aeruginosa* infections.

A number of schemes are used to distinguish between the different serotypes of *Pseudomonas aeruginosa.* The generally accepted International Antigenic Typing Scheme (IATS) is based on the serological properties of the O-antigen-specific lipopolysaccharide-(LPS-) associated carbohydrates, and at least twenty distinct O-antigen-serotypes have been described. It has been shown that the majority of nosocomial *Pseudomonas aeruginosa* infections are associated with certain serotypes. Although the incidence of serotypes varies among countries, epidemiological reviews show that the serotypes IATS-O1, IATS-06, IATS-O11 and serogroup 2 (IATS-O2, IATS-O5, IATS-O16) are most prevalent and account for up to 70% of the clinical *Pseudomonas aeruginosa* isolates.

In order to treat patients suffering from *Pseudomonas aeruginosa* infections with O-antigen-specific and as such serotype-specific antibodies it is a fundamental need to determine the serotype of the infectious *Pseudomonas aeruginosa* strain prior to antibody treatment. For detection of *Pseudomonas aeruginosa* infections, diagnostic culture is considered the gold standard, but up to 2 days are required for detecting *Pseudomonas* by means of traditional culture-based methods. Unfortunately the shape, size and morphology of the bacterial colony after culturing do not allow the identification of the serotype. Serological methods have significant failure rates for unambiguous identification of the serotype. For example, slide agglutination-based serotype determination is a time consuming method and the results accomplished with these methods are strongly relying on experience and interpretation of the examiner and are further limited by self-agglutinating or non-agglutinating *Pseudomonas aeruginosa* strains.

The most reliable serotyping kits on the market have a failure rate of approximately 30%, where the *Pseudomonas aeruginosa* strains are classified as 'non typeable' due to self-agglutinating or non-agglutinating strains. As such the standard microbiological analysis has only limited capacity to reliably identify *Pseudomonas aeruginosa* and the related serotype within a reasonable time frame. In case a patient suffers from an infection caused by an antibiotic-resistant *Pseudomonas aeruginosa* strain, the time window for serotype identification is very critical, as mortality is strongly increasing with continuity of infection.

Consequently, methods of fast serotype identification are of high medical need in order to reduce the time between diagnosis and serotype-specific antibody treatment.

Recently, the molecular detection by means of biochip, microarray or PCR-based techniques has become a common procedure for the rapid identification of *Pseudomonas aeruginosa* and other bacterial species causing nosocomial infections. In the protocols of these methods genes containing unique, species-specific signature sequences are used. Many PCR-based methods described to date are targeting phylogenetic and functional genes by using oligonucleotides specifically annealing to regions of, e.g., the 16S rRNA or 23S rRNA. However, with these species-specific assays an identification of a certain serotype is not possible.

US20080026370 discloses oligonucleotide probes, useful for genotyping and pathotyping of *Pseudomonas aeruginosa* by means of hybridization assays on a biochip or microarray. A method for serotyping is not disclosed.

WO9741234 discloses the characterization of various genes belonging to the LPS O-antigen gene cluster of *Pseudomonas aeruginosa.* Provided is a method for detecting the serotypes O1 to 020 via polymerase chain reaction (PCR) in a sample comprising treating the sample with a primer which is capable of amplifying nucleic acid molecules comprising nucleotide sequences encoding PsbM (WbpM), or PsbN (WbpN). Specific oligonucleotide sequences to be used for reliable differentiation of various *Pseudomonas aeruginosa* serotypes are not disclosed.

Hence, the methods known in the art for identifying a particular *Pseudomonas aeruginosa* serotype show serious drawbacks:

Microbiological analysis of *Pseudomonas aeruginosa* serotype is time consuming and limited by self-agglutinating or non-agglutinating *Pseudomonas aeruginosa* strains. The known molecular methods for genotyping and serotyping of bacterial strains in general can not be used for reliable identification and differentiation within a bacterial species. Although some genes share homology between various species such as the ribosomal genes (16S rDNA, 23 S rDNA), it is not possible to use conserved oligonucleotide sequences representing these genes for reliable identification and differentiation of such serotypes within one bacterial species.

Thus, there is a high medical need for methods allowing fast and reliable detection of *Pseudomonas aeruginosa* serotypes which are specific for the bacterial species and for the most prevalent serotypes of *Pseudomonas aeruginosa.* In order to distinguish between bacterial colonization (≤10³⁻⁶ cfu/ml) and infection (>10⁶ cfu/ml) bacterial loads of *Pseudomonas aeruginosa* need to be determined over a range of at least 6 orders of magnitude. As such there is further a need for a reliable detection assay of *Pseudomonas aeruginosa* serotypes over a broad range of template concentrations.

Simultaneous detection of several *Pseudomonas aeruginosa* serotypes (preferably IATS-O1, IATS-O6, IATS-O11 and serogroup 2) from a single reaction is technically demanding, as primer and probe pairs of the individual assays influence each other, frequently leading to clinically insufficient detection limits.

Surprisingly, it has been found that the oligonucleotides and the assay and kit using the oligonucleotides according to the present invention allow a reliable and fast identification of *Pseudomonas aeruginosa* species and serotypes using serotype-specific primers.

### Description of the Invention

Accordingly, the technical problem underlying the present invention is to provide a fast, accurate, sensitive and reliable method for specific determination of the clinically most prevalent *Pseudomonas aeruginosa* serotypes.

This problem is solved by providing oligonucleotides, assays and kits using the oligonucleotides as defined in the following:

According to the present invention, an assay for serotype-specific detection of *Pseudomonas aeruginosa* in a sample is provided comprising the steps of:
a) annealing at least one pair of *Pseudomonas aeruginosa* serotype-specific primers to a target nucleic acid in a sample, wherein the at least one pair of *Pseudomonas aeruginosa* serotype-specific primers is selected from the group consisting of:
   (i) a first oligonucleotide primer comprising at least 10 consecutive nucleotides of the sequence shown in SEQ ID No 1 and a second oligonucleotide primer comprising at least 10 consecutive nucleotides of the sequence shown in SEQ ID No 2,
   (ii) a first oligonucleotide primer comprising at least 10 consecutive nucleotides of the sequence shown in SEQ ID No 3 and a second oligonucleotide primer comprising at least 10 consecutive nucleotides of the sequence shown in SEQ ID No 4,
   (iii) a first oligonucleotide primer comprising at least 10 consecutive nucleotides of the sequence shown in SEQ ID No 5 and a second oligonucleotide primer comprising at least 10 consecutive nucleotides of the sequence shown in SEQ ID No 6, and
   (iv) a first oligonucleotide primer comprising at least 10 consecutive nucleotides of the sequence shown in SEQ ID No 7 and a second oligonucleotide primer comprising at least 10 consecutive nucleotides of the sequence shown in SEQ ID No 8;
b) amplifying the target nucleic acid, and
c) detecting the amplified target nucleic acid.

The term "*Pseudomonas aeruginosa* serotype" as used herein means the presence of a specific O-antigen component of *Pseudomonas aeruginosa* outer carbohydrate lipopolysaccharide (LPS) being responsible for serotype specificity. The outer carbohydrate layer, in particular the O-antigen, of *Pseudomonas aeruginosa* varies markedly in different isolates of these bacteria. At least 20 different serotypes have been described based on the differences of the O-antigens (Rivera et al., 1992). The most prevalent *Pseudomonas aeruginosa* serotypes are serotypes IATS-O1, IATS-O6, IATS-O11 and serogroup 2 (IATS-O2, IATS-05, IATS-016).

The term "serotype-specific detection" as used herein means the detection and identification of one or more of the most prevalent *"Pseudomonas aeruginosa* serotypes" as defined above. According to the present invention, the detection is performed by analyzing the sample of interest by amplification techniques. A number of such amplification techniques including polymerase chain reaction (PCR) or ligase chain reaction (LCR) based technology is described in the prior art. Use of any such amplification techniques is a routine task for the skilled person and they represent suitable examples of herein relevant amplification techniques. The preferred gene amplification technique is PCR, more preferably real-time PCR, most preferred a multiplex real-time PCR or LightCycler-based real-time (multiplex) PCR.

The term "primer", "oligonucleotide primer" or "primer pair" as used herein refers to short oligonucleotides (typically 10-50 bp, preferably 15-35 bp) which are used in the amplification techniques mentioned before to amplify the DNA target sequence. The primers according to the invention are molecules containing at least 10, 11, 12, 13, 14, 15, 16, 17, 18, 19, 20, 21, 22, 23, 24, 25, 26, 27, 28, 29, 30, 31, 32, 33, 34 or 35 consecutive nucleotides of any of the nucleotide sequences shown in SEQ ID Nos 1 to 8. The primers or oligonucleotide primers as defined herein are produced synthetically using techniques known in the art, for example phosphotriester and phosphodiester methods (Gait et al., 1980), or automated techniques (Conolly, 1987).

The term "*Pseudomonas aeruginosa* serotype-specific primer" as used herein means primers capable of acting as a point of initiation of synthesis when placed under conditions which permit amplification of a nucleic acid sequence, which corresponds to or is complementary to the DNA coding for a serotype-specific target, e.g. an O-antigen locus. Conditions allowing the synthesis of a primer extension product include the presence of nucleotide substrates, an agent for polymerization such as DNA polymerase and a suitable temperature and pH. Preferably, the primers are nucleic acid sequences that do not undergo base pairing with other copies of the primer, and do not form a hair pin configuration. The primer length is between about 10-50 nucleotides, preferably about 15 - 35 nucleotides.

The present invention provides an assay as defined herein above wherein the step of detecting further comprises the step of hybridizing at least one pair of *Pseudomonas aeruginosa* serotype-specific hybridization probes to a sequence internal to the sequences where the at least one *Pseudomonas aeruginosa* serotype-specific primer pair anneals that was selected for performing the annealing step a) of the assay defined herein above. Hybridization techniques are known in the art and are described for example in Sambrook J, Fritsch EF, Maniatis T. in: Molecular Cloning, A Laboratory Manual, 1989 (Nolan C, Ed.), Cold Spring Harbor Laboratory Press, Cold Spring Harbor, NY.

According to a preferred embodiment, the at least one pair of *Pseudomonas aeruginosa* serotype-specific hybridization probes that hybridizes internal to the sequences where the at least one *Pseudomonas aeruginosa* serotype-specific primer pair anneals that was selected for performing the annealing step a) of the assay defined above, is selected from the group consisting of:
(i) a first oligonucleotide probe comprising at least 10 consecutive nucleotides of the sequence shown in SEQ ID No 9 and a second oligonucleotide probe comprising at least 10 consecutive nucleotides of the sequence shown in SEQ ID No 10,
(ii) a first oligonucleotide probe comprising at least 10 consecutive nucleotides of the sequence shown in SEQ ID No 11 and a second oligonucleotide probe comprising at least 10 consecutive nucleotides of the sequence shown in SEQ ID No 12,
(iii) a first oligonucleotide probe comprising at least 10 consecutive nucleotides of the sequence shown in SEQ ID No 13 and a second oligonucleotide probe comprising at least 10 consecutive nucleotides of the sequence shown in SEQ ID No 14, and
(iv) a first oligonucleotide probe comprising at least 10 consecutive nucleotides of the sequence shown in SEQ ID No 15 and a second oligonucleotide probe comprising at least 10 consecutive nucleotides of the sequence shown in SEQ ID No 16.

The term "probe", "oligonucleotide probe" or "hybridization probe" as used herein means any nucleotide sequence that is used to detect amplified target nucleotide sequences by hybridization. The probe according to the invention refers to short oligonucleotides (typically 10-50 bp, preferably 15-35 bp). The probe as used herein is a molecule comprising at least 10, 11, 12, 13, 14, 15, 16, 17, 18, 19, 20, 21, 22, 23, 24, 25, 26, 27, 28, 29, 30, 31, 32, 33, 34 or 35 consecutive nucleotides of the nucleotide sequences shown in any of SEQ ID Nos 9 to 16. The probe or oligonucleotide probe, may be produced synthetically using techniques known in the art. The probe may be linked, preferably covalently linked, to at least one detectable label which allows detection of the amplified products. Such "oligonucleotide probes" represent suitable hybridization probes, if they are specific for a serotype and hybridize to sequences internal to the sequences where the first primer set anneals.

The term "detectable label" as used herein does not exhibit any particular limitation. The detectable label may be selected from the group consisting of radioactive labels, luminescent labels, fluorescent dyes, compounds having an enzymatic activity, magnetic labels, antigens, and compounds having a high binding affinity for a detectable label. For example, fluorescent dyes linked to a probe may serve as a detection label, e.g. in a real-time PCR. Suitable radioactive markers are P-32, S-35, I-125, and H-3, suitable luminescent markers are chemiluminescent compounds, preferably luminol, and suitable fluorescent markers are preferably dansyl chloride, fluorcein-5-isothiocyanate, and 4-fluor-7-nitrobenz-2-aza-1,3 diazole, suitable enzyme markers are horseradish peroxidase, alkaline phosphatase, α-galactosidase, acetylcholinesterase, or biotin.

"Real-time PCR", relates to a single step closed tube method that is amenable to automated setup and data analysis. According to a preferred embodiment of the invention, LightCycler-based real-time PCR is used. Real-time PCR based procedures have the potential to incorporate primary diagnosis and target quantification.

In a preferred embodiment, a particular set of oligonucleotide sequences used in the amplification step ("oligonucleotide primers") is combined with a particular set of oligonucleotide sequences ("oligonucleotide probes"), in order to further increase specificity. In a most preferred embodiment, the primer and probe pairs are selected from one or more of the following pairs of oligonucleotide primer and probe sequences:
(i) the oligonucleotide primers having the sequence shown in SEQ ID NO: 1 and 2, and the oligonucleotide probes having the sequence shown in SEQ ID NO: 9 and 10;
(ii) the oligonucleotide primers having the sequence shown in SEQ ID NO: 3 and 4, and the oligonucleotide probes having the sequence shown in SEQ ID NO: 11 and 12;
(iii) the oligonucleotide primers having the sequence shown in SEQ ID NO: 5 and 6, and the oligonucleotide probes having the sequence shown in SEQ ID NO: 13 and 14; and
(iv) the oligonucleotide primers having the sequence shown in SEQ ID NO: 7 and 8, and the oligonucleotide probes having the sequence shown in SEQ ID NO: 15 and 16.

In a preferred embodiment of the invention, the step of detecting the *Pseudomonas aeruginosa* serotype is performed by
- quantification analysis, wherein the bacterial load can be determined by comparison with a standard curve of known bacterial concentrations;
- monitoring of amplification curves, wherein successful amplification of a PCR product can be observed by the increase of signal intensity;
- and/or melting curve analysis, wherein the PCR amplification results in the synthesis of a product with individual melting temperature.

The hybridization probes of the present invention provide the maximum level of specificity and allow a) bacterial load quantification by comparison with standard curve, b) observing serotype-specific amplification on-line or c) identifying the serotype by melting curve analysis.

The assay of the present invention has many practical applications. For example, the assay may be used to detect the most prevalent *Pseudomonas aeruginosa* serotypes IATS-O1, IATS-O6, IATS-O11 and serogroup 2 (IATS-O2, IATS-O5, IATS-016) individually or simultaneously in any medical, veterinarian or environmental sample suspected of containing *Pseudomonas aeruginosa* in a broad range of copy numbers. For specific detection of one serotype in a sample of interest 2 sets of oligonucleotide sequences are used: 2 primers (forward =fwd and reverse=rev) and 2 probes. In a preferred embodiment of the invention an assay is provided, wherein two or more *Pseudomonas aeruginosa* serotypes are detected simultaneously, also referred to as multi-valent or multiplex assay. For specific detection of e.g. the 4 most prevalent *P.aeruginosa* serotypes mentioned above, 4x 2 primers and 4x 2 probes are used in one assay (4-valent assay). Preferably, the *Pseudomonas aeruginosa* serotypes to be detected are the most prevalent serotypes IATS-01, IATS-06, IATS-011 and serogroup 2, wherein serogroup 2 contains serotypes IATS-02, IATS-05 and IATS-016. Primers (forward=fwd and reverse=rev) and probes may be used simultaneously in one step or consecutively in two separate steps.

Most preferred is the use of the oligonucleotide sequences as defined herein in a 4-valent Light Cycler assay. The term "4-valent LightCycler assay" means an assay that allows simultaneous detection of 4 *Pseudomonas aeruginosa* serotypes, in particular the *4-valent* LightCycler assay allows the parallel detection of the four most prevalent serotypes IATS-01, IATS-06, IATS-011 and serogroup 2 of *Pseudomonas aeruginosa.* The assay further shows very high specificity and sensitivity in a single reaction. However, the amount of non-typeable strains can be reduced by the 4-valent assay, and the 4-valent LightCycler assay is successfully validated for direct measurement from clinical broncho-alveolar lavage (BAL) samples. In LightCycler-based real-time PCR, amplification and detection occur in closed glass capillaries, preventing amplicon cross-contamination. The LightCycler achieves high-speed thermal cycling through fan-driven air rather than heat block conduction. Results of the 4-valent *Pseudomonas aeruginosa* serotyping test may thus be obtained in approximately one hour.

Detection of *Pseudomonas aeruginosa* according to the invention, especially by the above described 4-valent serotyping test may be performed on isolated bacterial DNA, or directly from clinical samples like sputum, broncho-alveolar lavage or tracheal aspiration, usually after dilution in ultrapure H₂O. Preferred are samples directly obtained from a lung lavage of a human such as a human patient with a pulmonary disorder. Clinical samples might also include bodily materials such as blood, urine, tissues and the like. Typically the samples may be taken from wound, burn, lung, and urinary tract infections of humans or mammals. The serotype of *Pseudomonas aeruginosa* may also be detected from food, soil or water samples.

In *Pseudomonas aeruginosa* infected pneumonia patients bacterial loads ranging from 10 cfu/ml up to 10⁹ cfu/ml can be routinely found. Depending on the method of obtaining respiratory secretions (e.g. broncho-alveolar lavage, endotracheal specimen, etc.), different interpretative cut-off points for *Pseudomonas* colonization or acute infection have been defined in clinical daily routine. These cut-off points are ranging from ≤10³⁻⁶ cfu/ml for bacterial colonization and >10⁶ cfu/ml for infection, e.g. pneumonia. Hence bacterial loads need to be determined over a range of at least 6 orders of magnitude. Thus, it appears desirable to provide a diagnostic method that allows reliable *Pseudomonas aeruginosa* serotype identification over a broad range of template concentrations.

Surprisingly, according to the invention the detection of the *Pseudomonas aeruginosa* serotype can be carried out in a broad range of template concentrations in the sample. In one embodiment of the invention, the bacterial loads in the sample are detectable in the range of 10 cfu/ml to 10⁹ cfu/ml, or in a range of 10² cfu/ml up to 10⁸ cfu/ml, or 10³ cfu/ml up to 10⁸ cfu/ml, or 10⁴ cfu/ml up to 10⁸ cfu/ml.

According to a further embodiment of the invention the assay is species-specific. Unless otherwise indicated, the term "species-specific" is used herein to indicate specificity for the species *Pseudomonas aeruginosa.* In particular, "species-specific detection" means that frequently occurring microorganisms (bacteria, fungi or viruses) other than *Pseudomonas aeruginosa,* such as *Acinetobacter baumannii, Chlamydophila pneumoniae, Enterobacter aerogenes, Enterobacter cloacae, Escherichia coli, Klebsiella oxytoca, Klebsiella pneumoniae, Proteus mirabilis, Serratia marcescens, Stenotrophomonas maltophilia, Actinobacillus actinomycetemcomitans, Aeromonas hydrophilia, Bacterioides fragilis, Bartonella henselae, Bordetella pertussis, Borrelia burgdorferi, Burkholderia cepacia, Campylobacter jejuni, Cardiobacterium hominis, Haemophilus influenza, Legionella pneumophila, Listeria monocytogenes, Moraxella catarrhalis, Morganella morganii, Neisseria meningitidis, Pantoea agglomerans, Porphyromonas gingivalis, Prevotella denticola, Proteus vulgaris, Yersinia enterocolitica, Enterococcus faecalis, Enterococcus faecium, Staphylococcus aureus, Staphylococcus epidermidis, Staphylococcus haemolyticus, Streptococcus pneumoniae, Streptococcus agalactiae, Streptococcus pyogenes, Streptococcus mitis, Bacillus cereus, Clostridium perfringens, Corynebacterium jeikeium, Gemella haemolysans, Histoplasma capsulatum, Mycobacterium fortuitum, Mycobacterium tuberculosis, Mycoplasma pneumoniae, Peptostreptococcus magnus, Propionibacterium acnes, Aspergillus fumigatus, Candida albicans, Candida glabrata, Candida krusei, Candida parapsilosis, Candida tropicalis, Cryptococcus neoformans, Adenoviruses, Herpesviruses, Orthomyxoviruses, Paramycxoviruses, and Picornaviruses* are not detected with the assay, kit or oligonucleotides of the invention.

Further provided is a pair of oligonucleotides capable of specifically determining one or more *Pseudomonas aeruginosa* serotypes selected from the group of IATS 01, S2 (serogroup 2 comprising IATS-02, IATS-O5, IATS-O16), IATS 06, and IATS O11 as defined above, wherein said pair of first and second oligonucleotides comprise at least 10, 11, 12, 13, 14, 15, 16, 17, 18, 19, 20, 21, 22, 23, 24, 25, 26, 27, 28, 29, 30, 31, 32, 33, 34 or 35 consecutive nucleotides of the nucleotide sequences shown in SEQ ID Nos.: 1 and 2, 3 and 4, 5 and 6, 7 and 8, 9 and 10, 11 and 12, 13 and 14, or 15 and 16. In particular, the present invention provides a pair of oligonucleotides selected from the group consisting of:
a) a first oligonucleotide comprising at least 10 consecutive nucleotides of the sequence shown in SEQ ID No 1 and a second oligonucleotide comprising at least 10 consecutive nucleotides of the sequence shown in SEQ ID No 2,
b) a first oligonucleotide comprising at least 10 consecutive nucleotides of the sequence shown in SEQ ID No 3 and a second oligonucleotide comprising at least 10 consecutive nucleotides of the sequence shown in SEQ ID No 4,
c) a first oligonucleotide comprising at least 10 consecutive nucleotides of the sequence shown in SEQ ID No 5 and a second oligonucleotide comprising at least 10 consecutive nucleotides of the sequence shown in SEQ ID No 6,
d) a first oligonucleotide comprising at least 10 consecutive nucleotides of the sequence shown in SEQ ID No 7 and a second oligonucleotide comprising at least 10 consecutive nucleotides of the sequence shown in SEQ ID No 8;
e) a first oligonucleotide comprising at least 10 consecutive nucleotides of the sequence shown in SEQ ID No 9 and a second oligonucleotide comprising at least 10 consecutive nucleotides of the sequence shown in SEQ ID No 10,
f) a first oligonucleotide comprising at least 10 consecutive nucleotides of the sequence shown in SEQ ID No 11 and a second oligonucleotide comprising at least 10 consecutive nucleotides of the sequence shown in SEQ ID No 12,
g) a first oligonucleotide comprising at least 10 consecutive nucleotides of the sequence shown in SEQ ID No 13 and a second oligonucleotide comprising at least 10 consecutive nucleotides of the sequence shown in SEQ ID No 14, and
h) a first oligonucleotide comprising at least 10 consecutive nucleotides of the sequence shown in SEQ ID No 15 and a second oligonucleotide comprising at least 10 consecutive nucleotides of the sequence shown in SEQ ID No 16.

The oligonucleotides of the invention have the following technical characteristics shown in Table 1 :

**Table 1**

| **SEQ ID NO** | **Tm value (in °C)*¹** | **GC content (in %)** |
|---|---|---|
| 1 | 60.0 | 42.9 |
| 2 | 56.0 | 55.6 |
| 3 | 54.0 | 58.8 |
| 4 | 54.0 | 58.8 |
| 5 | 56.0 | 47.4 |
| 6 | 60.0 | 50.0 |
| 7 | 52.0 | 52.9 |
| 8 | 60.0 | 42.9 |
| 9 | 64.0 | 60.0 |
| 10 | 95.0 | 44.1 |
| 11 | 50.0 | 66.7 |
| 12 | 95.0 | 42.9 |
| 13 | 52.0 | 62.5 |
| 14 | 52.0 | 62.5 |
| 15 | 66.0 | 57.1 |
| 16 | 86.0 | 43.3 |
| 17 | 95.0 | 44.1 |

| | | |
|---|---|---|
| *¹ Melting point calculation following the "2+4 rule" (A/T nucleotides = 2°C, G/C nucleotides = 4 °C) | | |

The oligonucleotide sequences according to the invention are able to specifically determine Pseudomonas serotype IATS 01, S2 (serogroup 2 comprising IATS-O2, IATS-O5, IATS-O16), IATS 06, IATS O11. Preferred oligonucleotides used in accordance with the invention are primers and probes shown in the Table 2 below:

**Table 2**

| **Oligonucleotide Sequence/ Type** | **IATS Serotype** | **Name** | **Label** | **5' - 3' Sequence** | **SEQ ID NO** |
|---|---|---|---|---|---|
| Primer | O1 | O1-fwd | - | TAC GCC GTG AAG ATA GAA TTG | 1 |
| | | O1-rev | - | TCT CCT CTA AGC GCC TTG | 2 |
| | S2 | S2-fwd | - | TTC ATG GGT GAT GCG GG | 3 |
| | | S2-rev | - | TCC GGC GGA TCA GAG TA | 4 |
| | O6 | O6-fwd | - | TGT ACT GGC TTG GAG GTT T | 5 |
| | | O6-rev | | ATC GGA ACC TAC ACC ACT GA | 6 |
| | O11 | O11-fwd | - | TAG TGC AGC CTT GGT CT | 7 |
| | | O11-rev | - | CGA TCC CAT CCA TGA AGT TAT | 8 |
| Probe | O1 | O1-Fluo | Fluo | TGG GCA GGT ATC TGA GCA GC | 9 |
| | | O1-Red640 | Red640 | TCG GCT ATC ATG AAC GGT AGC TTG ATG TAT ATG C | 10 |
| | S2 | S2-Fluo | Fluo | CCC TCG CTG TTC TGG | 11 |
| | | S2-Red610 | Red610 | GTT GAT ATT GCT GGG AGT GTT CAT CGT TGA TGC AA | 12 |
| | O6 | O6-Fluo | Fluo | CAG CGT TGA GGC TGT G | 13 |
| | | O6-Red705 | Red705 | TGT GTG CGT TGG CGC T | 14 |
| | O11 | O11-Fluo | Fluo | TTG GTG TCA CTT GGG ACC TGG | 15 |
| | | O11-Red670 | Red670 | TGG TTC GGA GGA CTT CTC TTT GCT TTC TAT | 16 |
| | IC | IC-Red610 | Red610 | AGC CGA TAG TAC TTG CCA TCG AAC TAC ATA TAC G | 17 |

The combined action of primers as defined herein is capable of serotype-specific amplification of a suitable target gene for subsequent or simultaneous serotype specific detection by corresponding hybridization probes.

The target genes that have been used in accordance with the present invention for the design of serotype-specific primer/probe pairs belonging to the LPS O-antigen gene cluster of *Pseudomonas aeruginosa* are the genes named wzz and GtGr4. The sequence of the target genes for Pseudomonas serotype IATS 01, S2 (serogroup 2 comprising IATS-O2, IATS-O5, IATS-O16), IATS 06, IATS O11 wzz and GtGr4 is shown in SEQ ID NO: 18 to 21 of the sequence listing.

The target gene for *Pseudomonas aeruginosa* serotype IATS-O1 is shown in SEQ ID NO: 18: and can be found in NCBI Accession AF498400; Version AF498400.1; ORF_4; Region:1284-2321 ORF_4; wzz; similar to chain length determinant protein.
The target gene for Serogroup 2 (*Pseudomonas aeruginosa* serotype IATS-O2, IATS-05, and IATS-O16; identical sequence for all three serotypes) is shown in SEQ ID NO: 19 and can be found in NCBI Accession AF498412; Version AF498412.1; ORF_19; Region: 18769 to19788 ORF_19; GtGr4; similar to Glycosyl transferase group 4; potential multiple membrane spanning domains.

The target gene for *Pseudomonas aeruginosa* serotype IATS-06 is shown in SEQ ID NO: 20 And can be found in NCBI Accession AF498417; Version AF498417.1; ORF_14; Region: 12654-13694, ORF_14; GtGr4; similar to Glycosyl transferase group 4; potential multiple membrane spanning domains).

The target gene for *Pseudomonas aeruginosa* serotype IATS-O11 is shown in SEQ ID NO: 21 and can be found in NCBI Accession AF498402; Version AF498402.1; ORF_13; Region: 11073-12098, ORF_13; GtGr4; similar to Glycosyl transferase group 4; potential multiple membrane spanning domains.

The LPS-O-antigen cluster has been identified as region with high genetic diversity and it has been speculated that the sequences identified and determined provide an opportunity to develop DNA sequence-based PCR methods to differentiate between the *Pseudomonas aeruginosa* serotypes (Raymond et al., 2002). Altogether eleven groups of O-antigen gene clusters have been defined by Raymond and coauthors from the twenty *Pseudomonas aeruginosa* serotypes. Although the authors claimed that each gene cluster was shown to be highly divergent from one another at the DNA sequence level, the inventors experienced that the DNA differences of the majority of the potential target genes were too small to allow a serotype-specific oligonucleotide design. Furthermore, it turned out that even in the few genes possessing high genetic diversity most of the designed oligonucleotides could not be used for reasons of missing serotype-specificity, insufficient sensitivity, low signal intensity or strong signal variation in dependence of the applied template concentration.

The reagents suitable for applying the assays of the invention may be packaged into convenient kits providing the necessary materials, packaged into suitable containers.

Such kits may include all the reagents required to detect the *Pseudomonas aeruginosa* serotypes, preferably IATS-O1, IATS-O6, IATS-O11 and serogroup 2 (IATS-O2, IATS-05, IATS-016) in a sample by means of the methods described herein, and optionally suitable supports useful in performing the methods of the invention. Preferably, a kit according to the invention also includes appropriate positive and/or internal control target nucleic acid sequences as well as nucleic acid sequences or H₂O used as negative control.
The assays and kits of the present invention have many practical applications. For example, the methods and kits of the present invention may be used to detect the *Pseudomonas aeruginosa* serotypes IATS-O1, IATS-O6, IATS-O11 and serogroup 2 (IATS-O2, IATS-O5, IATS-O16) simultaneously in any medical, veterinarian or environmental sample suspected of containing *Pseudomonas aeruginosa.*

The kits may further provide reagents and controls for virulence and resistance markers.

According to a further preferred embodiment, the present invention provides a kit for performing the assay for the serotype-specific detection of *Pseudomonas aeruginosa* as defined. The kit comprises at least one *Pseudomonas aeruginosa* serotype-specific primer pair selected from the group consisting of:
i. a first oligonucleotide primer comprising at least 10 consecutive nucleotides of the sequence shown in SEQ ID No 1 and a second oligonucleotide primer comprising at least 10 consecutive nucleotides of the sequence shown in SEQ ID No 2,
ii. a first oligonucleotide primer comprising at least 10 consecutive nucleotides of the sequence shown in SEQ ID No 3 and a second oligonucleotide primer comprising at least 10 consecutive nucleotides of the sequence shown in SEQ ID No 4,
iii. a first oligonucleotide primer comprising at least 10 consecutive nucleotides of the sequence shown in SEQ ID No 5 and a second oligonucleotide primer comprising at least 10 consecutive nucleotides of the sequence shown in SEQ ID No 6, and
iv. a first oligonucleotide primer comprising at least 10 consecutive nucleotides of the sequence shown in SEQ ID No 7 and a second oligonucleotide primer comprising at least 10 consecutive nucleotides of the sequence shown in SEQ ID No 8; and reagents required for annealing, and optionally reagents for amplification and/or instructions for the serotype-specific detection of *Pseudomonas aeruginosa.*

Reagents useful in such kits are enzyme solutions containing DNA or RNA amplifying enzymes, enzyme solutions containing decontamination enzymes, buffer solutions containing Mg²⁺, dNTP mixes and/or the primer and hybridization probe mixture, appropriate positive and/or internal control as well as nucleic acid sequences or H₂O used as negative control.

In a preferred embodiment of the invention, the kit further comprises at least one pair of *Pseudomonas aeruginosa* serotype-specific hybridization probes that hybridizes to a sequence internal to the sequences where the at least one serotype-specific primer pair anneals.

Preferably, the at least one pair of *Pseudomonas aeruginosa* serotype-specific hybridization probes are selected from the group consisting of:
(i) a first oligonucleotide probe comprising at least 10 consecutive nucleotides of the sequence shown in SEQ ID No 9 and a second oligonucleotide probe comprising at least 10 consecutive nucleotides of the sequence shown in SEQ ID No 10,
(ii) a first oligonucleotide probe comprising at least 10 consecutive nucleotides of the sequence shown in SEQ ID No 11 and a second oligonucleotide probe comprising at least 10 consecutive nucleotides of the sequence shown in SEQ ID No 12,
(iii) a first oligonucleotide probe comprising at least 10 consecutive nucleotides of the sequence shown in SEQ ID No 13 and a second oligonucleotide probe comprising at least 10 consecutive nucleotides of the sequence shown in SEQ ID No 14, and
(iv) a first oligonucleotide probe comprising at least 10 consecutive nucleotides of the sequence shown in SEQ ID No 15 and a second oligonucleotide probe comprising at least 10 consecutive nucleotides of the sequence shown in SEQ ID No 16.

Most preferred, the kit of the invention contains one or more primer and probe pairs selected from the following pairs of oligonucleotide sequences:
(i) the oligonucleotide primers having the sequence shown in SEQ ID NO: 1 and 2, and the oligonucleotide probes having the sequence shown in SEQ ID NO: 9 and 10;
(ii) the oligonucleotide primers having the sequence shown in SEQ ID NO: 3 and 4, and the oligonucleotide probes having the sequence shown in SEQ ID NO: 11 and 12;
(iii) the oligonucleotide primers having the sequence shown in SEQ ID NO: 5 and 6, and the oligonucleotide probes having the sequence shown in SEQ ID NO: 13 and 14; and
(iv) the oligonucleotide primers having the sequence shown in SEQ ID NO: 7 and 8, and the oligonucleotide probes having the sequence shown in SEQ ID NO: 15 and 16.

The kit of the invention may further contain reagents required to produce the amplified nucleic acid molecule or predetermined fragment thereof in the polymerase chain reaction, and means for assaying the amplified sequences.

The serotype-specific probe contained in the kit of the invention is labeled with a detectable marker. Preferably, the detectable marker is selected from the group consisting of: luminescent markers, fluorescent markers, radioactive markers and enzyme markers.

In a preferred embodiment, the kit according to the invention is capable of specifically detecting the *Pseudomonas aeruginosa* serotypes IATS-01, IATS-06, IATS-011 and serogroup 2, wherein serogroup 2 contains serotypes IATS-02, IATS-05 and IATS-016, simultaneously.

The present invention is further directed to the use of serotype specific antibodies for a more effective treatment of a *Pseudomonas aeruginosa* infection in patient group which was diagnosed for said specific *Pseudomonas aeruginosa* serotype.

The present invention provides the use of an antibody specific for one or more of *Pseudomonas aeruginosa* serotype IATS-01, IATS-06, IATS-011 and serogroup 2, wherein serogroup 2 contains serotypes IATS-02, IATS-05 and IATS-016, for the preparation of a medicament for the treatment of an *Pseudomonas aeruginosa* infection.

Accordingly, in a preferred embodiment the present invention provides the use of an antibody specific for one or more of *Pseudomonas aeruginosa* serotype IATS-01, IATS-06, IATS-011 and serogroup 2, wherein serogroup 2 contains serotypes IATS-02, IATS-05 and IATS-016, for the preparation of a medicament for the treatment of an *Pseudomonas aeruginosa* in a patient in whom said specific *Pseudomonas aeruginosa* serotype according to the assay of the invention has been detected.

In a further embodiment, the present invention provides an antibody specific for one or more of the *Pseudomonas aeruginosa* serotypes IATS-01, IATS-06, IATS-011 and serogroup 2, wherein serogroup 2 contains serotypes IATS-02, IATS-05 and IATS-016 for the use in treating *Pseudomonas aeruginosa* infection.

In a preferred embodiment, the present invention provides an antibody specific for one or more of the *Pseudomonas aeruginosa* serotypes IATS-01, IATS-06, IATS-011 and serogroup 2, wherein serogroup 2 contains serotypes IATS-02, IATS-05 and IATS-016 for the use in treating *Pseudomonas aeruginosa* infection in a patient, in whom said specific *Pseudomonas aeruginosa* serotype according to the assay of the invention has been detected.

The present invention further provides the use of the oligonucleotide primers or probes as defined herein above for serotype-specific detection of *Pseudomonas aeruginosa.*

The following examples illustrate the invention but are not intended to limit the scope of the present invention. Further embodiments will be apparent for the person skilled in the art when studying the specification and having regard to common general knowledge.

### BRIEF DESCRIPTION OF THE DRAWINGS

**Figure 1**: Figure 1 schematically shows the assay result of the 4-valent *Pseudomonas aeruginosa* serotyping test after positive serotype detection by LightCycler-based melting curve analysis.
**Figure 2**: Figure 2 shows the reproducibility of detected Tm values.
**Figure 3**: Figure 3 shows exemplary Tm values of non-functional primer/probe combinations.

### EXAMPLES

### MATERIAL AND METHODS

### LightCycler based 4-valent Pseudomonas aeruginosa serotyping test

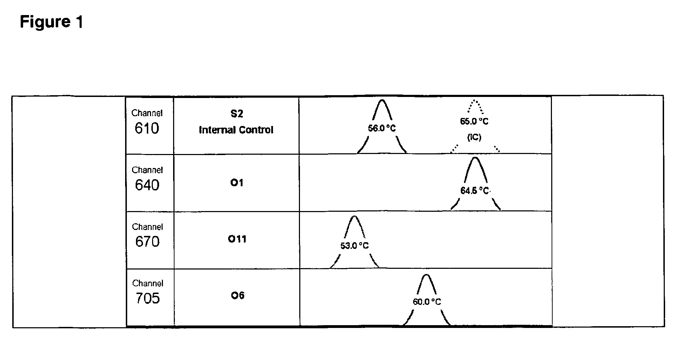

The 4-valent *Pseudomonas aeruginosa* serotyping test is capable of providing serotype-specific sequence confirmation of the amplified product through a function called melting curve analysis. At the end of each PCR run the temperature in the thermal chamber is slowly raised. During this process the fluorescence in each capillary is measured at frequent intervals. This allows the melting behavior of the amplified DNA to be monitored very closely. As soon as the temperature is raised fluorescence will decrease as a result of FRET (fluorescence resonance energy transfer) ceasing when one of the probes is released. For interpretation of melting curve analysis the first negative derivative of the sample fluorescence is plotted versus temperature and displayed as a melting temperature peak of each sample. The temperature at which probes are displaced can vary largely, depending on their sequence, length and GC content and even single nucleotide differences can change melting temperature. Thus, melting temperature profiles can be used to identify specific DNA products. By performing a melting curve analysis after performance of the 4-valent *Pseudomonas aeruginosa* serotyping test using the primers and probes according to the invention, the *Pseudomonas* serotype of the PCR product from an unknown clinical sample can be determined by comparing its melting temperature (Tₘ) with the Tₘ of the product resulting from amplification of the positive control. In the example shown in Figure 1, the serotype specific Tₘ values for *Pseudomonas aeruginosa* serotypes IATS-O1, IATS-O6, IATS-011, and serogroup 2 are depicted at exemplary detection channels.

Inherently, PCR is not amenable to extensive multiplexing and interrogating multiple genetic targets requires multiple reactions. In LightCycler based real-time PCR this obstacle is resolved by an optical unit, which is capable of measuring fluorescence in separate channels simultaneously, thereby allowing analysis of more than one target sequence from a single sample. To enable simultaneous detection of several *Pseudomonas aeruginosa* serotypes (preferably IATS-O1, IATS-06, IATS-O11 and serogroup 2) from a single reaction, multiple probes each labelled with an individual color and multiple primers may be used in the 4-valent *Pseudomonas aeruginosa* serotyping test.

Such reactions provide a higher degree in complexity and thus it is crucial to determine suitable reaction conditions resulting in specific and reliable results. However such multiplex analysis is only specific and sensitive if the reaction parameters are carefully chosen. Criteria for assay design are e.g.
- the design of the primers
- the design of the (hybridization) probes
- target concentration
- non specific residual DNA in the sample to be analysed (contamination)
- sample preparation from different biological matrices (e.g. from broncho-alveolar lavage)

### Target gene identification

For target gene identification, the open reading frames (ORF) constituting the O-antigen gene loci of the different *Pseudomonas aeruginosa* serotypes were downloaded from genebank and compared by Clustal like alignment (Chenna *et al.,* 2003). In a first selection process, ORFs that are either not present in the O-antigen gene loci of the serotypes of question or that are present multiple times were rejected. In a second step, ORFs possessing insufficient sequence diversity and ORFs that are too small for design of primer/probe-based assays were excluded. Applying these selection criteria only four out of 28 ORFs seemed to be suitable for primer/probe design. Based on the genebank data of the Pseudomonas O-antigen gene clusters, two of the four remaining ORFs (Asparagine synthase and Oxidoreductase family NAD-binding Rossmann fold) were thought to be present in only a limited number of serotypes. However, after primer/probe design it turned out that serotype independent PCR amplification was observed from both genes. As a consequence it can be concluded that duplicates of these genes can be found outside the O-antigen gene locus of the respective serotypes and that the genes are thus not suited for serotype differentiation.

### Primer/Probe design

For serotype specific detection of *Pseudomonas aeruginosa* isolates specific primer/probe oligonucleotides are of need. For the design of such sequences the LightCycler Probe Design Software 2.0 (Roche Applied Science) was used. First, sequence data of the two remaining target genes (wzz, similar to chain length determinant protein and GtGr4, similar to glycosyltransferase group 4) were imported into the software followed by automated analysis and scoring of resulting primer/probe sequences according to the chosen design parameters.

For the primer/probe pairs described in the present invention several design presettings were applied: primer/probe sets for quantitative PCR, primer/probe sets for melting curve analysis, and primer/probe sets for multiplex PCR detection. In a next step the proposed primer/probe pairs were tested for potential cross-complementarities by direct submission to the NCBI website.

After completion of the design process, resulting nucleotide sequences were initially tested with only a very limited number of bacterial isolates. It turned out that measurement of particular *Pseudomonas aeruginosa* reference strains at different dilutions was the most meaningful experiment for first evaluation of the chosen primer/probe pairs. Under these conditions the vast majority of designed assays did neither produce reliable serotype detection signals nor enabled detection with sufficient sensitivity. However, reliable and stable serotype detection over a broad range of template concentrations is of high clinical relevance and as such an essential prerequisite for successful design of a diagnostic assay. Consequently, primer/probe pairs that did not meet these requirements were immediately rejected.

When summarizing the software-based primer/probe design process, many oligonucleotide sequences that fulfill the general requirements of good primer and probe design (e.g. no intra-molecular sequence homologies, single annealing sites on template, amplicon size, desired melting temperature, etc.) were suggested. Nevertheless, many of the proposed primer/probe pairs could not be used for reliable serotype identification because of too much variation in the detection signals, because of missing serotype specificity or because of insufficient sensitivity.

Primer/probe pairs of the four separate serotyping tests (IATS-O1, IATS-O6, IATS-011, and serogroup 2 detection) that fulfill all requirements concerning identical reaction conditions, serotype- and species-specificity as well as analytical sensitivity were combined in one reaction aiming to provide a multiplex assay that allows simultaneous detection of four *Pseudomonas aeruginosa* serotypes. At this stage the primer/probe pairs of several tests had to be refined beyond the software based design (e.g. serogroup 2 specific probes were optimized for spacing between the probe partners to increase signal intensity whereas IATS-O11 specific probes were refined by shortening and destabilization of one of the probes to prevent signal crosstalk between the different detection channels).

After final refinement the 4-valent format of the *Pseudomonas aeruginosa* serotyping assay enables reproducible detection of 10 to 10⁷ specific gene copies per reaction (corresponds to 10² to 10⁹ gene copies/ml). Such specific serotype identification may also be observed in the presence of up to 10⁶ gene copies per reaction (corresponds to 10⁸ gene copies/ml) of both unrelated *Pseudomonas aeruginosa* serotypes or DNA from other species (e.g. bacteria, fungi and viruses).

It will be appreciated that the primer/probes may contain non-complementary sequences provided that a sufficient amount of the primer/probes contains a sequence which is complementary to a nucleic acid molecule in the sample analysed or oligonucleotide fragment thereof, which is to be amplified. Restriction site linkers may also be incorporated into the primers allowing for digestion of the amplified products with the appropriate restriction enzymes facilitating cloning and sequencing of the amplified product.

The reaction conditions for (real-time) PCR are, generally, dependent upon primer length and composition, template concentration, the length of the DNA segment to be amplified, the properties of the thermostable DNA polymerase, and Magnesium (Mg ²⁺) concentration. Each of these parameters is readily determinable by one of ordinary skill in the art in accordance with conventional practices.

In most instances, the amplified DNA is detected by agarose or acrylamide gel electrophoresis, ethidium bromide staining and UV irradiation. To increase sensitivity of PCR or to provide faster results, one or both of the oligonucleotide primers and probes can be labeled as described above or a label can be incorporated into the amplified DNA during polymerization. Labeled DNA products may be analyzed on-line during amplification, thereby eliminating the need for result interpretation by gel electrophoresis.

### Example 1: Microbiological Samples

For development of the 4-valent *Pseudomonas aeruginosa* serotyping test sixteen reference strains and ninety four clinical isolates were used. Reference strains were obtained from either Institute Pasteur (Paris, France) or the Belgium bacteria collection BCCM (Gent, Belgium). Clinical isolates were obtained from University Hospitals Basle, Berne, Zurich (Switzerland) or Jena (Germany) and from a multinational clinical study on Cystic Fibrosis.

For comparison of the developed 4-valent *Pseudomonas aeruginosa* serotyping test with a commercially available agglutination test (BioRad), almost five hundred respiratory *Pseudomonas aeruginosa* isolates that were collected at approximately twenty hospitals in the United States, Germany, Greece, and Belgium were used.

### Example 2: Sample Preparation

### 2.1 Measurement of purified bacterial DNA

Each of the reference strains and the clinical isolates were grown over Night (oN) in 5 ml BHI media (BD Bioscience) at 37°C. DNA of the bacteria was isolated from bacterial cell pellets using the high pure PCR template preparation kit (Roche Diagnostics). Before application in PCR, DNA stock solutions of all DNA samples were normalized to 20 ng/µl From DNA stock solutions serially diluted working solutions were prepared by 1:10 dilution steps in ultrapure H₂O (Roche Diagnostics). Five µl of each of the DNA working solutions were applied as template solution.

### 2.2. Measurement from bacterial culture

Reference strains and clinical isolates were plated on BHI agar plates (BD Bioscience). After oN incubation at 37°C one inoculation loop of bacteria was resolved in 500 µl of ultrapure H₂O (Roche Diagnostics). Eight µl of each of the bacterial dilutions were applied as template solution.

### 2.3. Measurement from broncho-alveolar lavage (BAL) samples

Purified bacterial DNA was spiked in processed BAL samples at a concentration of 10³ and 10⁶ gene copies/reaction. BAL samples were obtained from University Hospital Berne. Before application in PCR, spiked BAL samples were diluted (1:3) in Sputasol (Oxoid) and solubilized by incubation for 30 min at 36°C. Spiked and solubilized BAL samples were finally diluted (1:100) in ultrapure H₂O (Roche Diagnostics). Eight µl of each of the processed BAL samples were applied as template solution.

### Example 3: Primer and Assay Design

### 3.1. Selection of target genes

**Table 3: Target genes that have been used for the design of serotype-specific primer/probe pairs.**

| **Assay** | **IATS Serotype** | **Target Gene and Locus Definition** | **Reference Sequence** | **Primer** / **Probes** | **SEQ ID NO:** |
|---|---|---|---|---|---|
| O1 | O1 | Wzz, | IATS O1 reference strain, ATCC 33348 | O1-fwd / O1-rev | 18 |
| | | similar to chain length determinant protein NCBI accession AF498400; ORF_4; region 1284-2321 | | O1-Fluo / O1-Red640 | |
| S2 | S2 | GtGr4, | IATS O2 reference strain, ATCC 33356 | S2-fwd / S2-rev | 19 |
| | | similar to glycosyltransferase group 4 NCBI accession AF498412; ORF_19; region 18769-19788 | | S2-Fluo / S2-Red610 | |
| O6 | O6 | GtGr4, | IATS O6 reference strain, ATCC 33354 | O6-fwd / O6-rev | 20 |
| | | similar to glycosyltransferase group 4 NCBI accession AF498417; ORF_14; region 12654-13694 | | O6-Fluo / O6-Red705 | |
| O11 | O11 | GtGr4, | IATS O11 reference strain, ATCC 33358 | O11-fwd / O11-rev | 21 |
| | | similar to glycosyltransferase group 4 NCBI accession AF498402; ORF_13; region 11073-12098 | | O11-Fluo / O11-Red670 | |

### 3.2. Design of primer and probe sequences

Primer/probe oligonucleotides were designed using the LightCycler Probe Design Software 2.0 (Roche Applied Science). Beyond software based design the serogroup 2 specific probes were optimized for spacing between the probe partners and the serotype O11 specific probes were refined by shortening and destabilization of the sensor probe.

**Table 4:**

| **4-valent *Pseudomonas aeruginosa* serotyping test - Primer and Probe sequences** For specific detection of one serotype in a sample of interest 2 sets of oligonucleotide sequences are used: 2 primers and 2 probes. For specific detection of e.g. the 4 most prevalent*Pseudomonas aeruginosa* serotypes mentioned above multiple primers and probes are used in one assay. | | | | | |
|---|---|---|---|---|---|
| **Typ** | **IATS Serotype** | **Name** | **Label** | **5' - 3' Sequence** | **Final Assay Concentration** |
| Primer | O1 | O1-fwd | - | TAC GCC GTG AAG ATA GAA TTG | 0.5 µM |
| | | 01-rev | - | TCT CCT CTA AGC GCC TTG | 0.7 µM |
| | S2 | S2-fwd | - | TTC ATG GGT GAT GCG GG | 0.5 µM |
| | | S2-rev | - | TCC GGC GGA TCA GAG TA | 0.5 µM |
| | O6 | 06-fwd | - | TGT ACT GGC TTG GAG GTT T | 0.5 µM |
| | | O6-rev | | ATC GGA ACC TAC ACC ACT GA | 0.5 µM |
| | O11 | O11-fwd | - | TAG TGC AGC CTT GGT CT | 0.5 µM |
| | | 011-rev | - | CGA TCC CAT CCA TGA AGT TAT | 0.7 µM |
| Probe | O1 | O1-Fluo | Fluo | TGG GCA GGT ATC TGA GCA GC | 0.15 µM |
| | | O1-Red640 | Red640 | TCG GCT ATC ATG AAC GGT AGC TTG ATG TAT ATG C | 0.3 µM |
| | S2 | S2-Fluo | Fluo | CCC TCG CTG TTC TGG | 0.15 µM |
| | | S2-Red610 | Red610 | GTT GAT ATT GCT GGG AGT GTT CAT CGT TGA TGC AA | 0.3 µM |
| | O6 | O6-Fluo | Fluo | CAG CGT TGA GGC TGT G | 0.3 µM |
| | | O6-Red705 | Red705 | TGT GTG CGT TGG CGC T | 0.3 µM |
| | O11 | O11-Fluo | Fluo | TTG GTG TCA CTT GGG ACC TGG | 0.2 µM |
| | | O11-Red670 | Red670 | TGG TTC GGA GGA CTT CTC TTT GCT TTC TAT | 0.2 µM |
| | IC | IC-Red610 | Red610 | AGC CGA TAG TAC TTG CCA TCG AAC TAC ATA TAC G | 0.3 µM |

### Example 4: PCR Conditions, Test components and Test Controls

**Table 5a**

| **4-valent *Pseudomonas aeruginosa* serotyping test - PCR conditions** | | | |
|---|---|---|---|
| **Experimental Step** | **No. of Cycles** | **Temperature (in °C)** | **Duration** |
| Denaturation | 1 | 95 | 10 min |
| Amplification | 45 | 95 | 10 sec |
| | | 55 | 10 sec |
| | | 72 | 15 sec |
| Melting | 1 | 95 | ≥ 1 sec |
| | | 40 | 30 sec |
| | | shift from 40 to 80 | 0.1 °C/sec |
| Cooling | 1 | 40 | 30 sec |

**Table 5b**

| **4-valent *Pseudomonas aeruginosa* serotyping test - Test components** For serotype-specific detection of *Pseudomonas aeruginosa* using the assay described in the present invention, primer and probe oligonucleotides as well as internal control and positive controls can be used in combination with the generic LightCycler FastStart DNA Master HybProbe Kit (Roche Diagnostics; Cat. No. 03 003248 001) and the decontamination enzyme LightCycler Uracil-DNA Glycosylase (Roche Diagnostics; Cat. No. 03 539 806 001). | | | |
|---|---|---|---|
| **Component** | **Content / Function** | **Source** | **Volume for 1 PCR reaction (in µl)** |
| LightCycler HybProbe Reaction Mix 1a | Enzyme solution containing FastStart Taq DNA Polymerase | LightCycler FastStart DNA Master HybProbe Kit, Roche Diagnostics | 0.3 |
| LightCycler HybProbe Reaction Mix 1b | Contains FastStart Taq DNA Polymerase reaction buffer and dNTP mix | LightCycler FastStart DNA Master HybProbe Kit, Roche Diagnostics | 1.7 |
| LightCycler HybProbe MgCl₂ stock solution | Contains Mg²⁺ | LightCycler FastStart DNA Master HybProbe Kit, Roche Diagnostics | 1.6 |
| LightCycler HybProbe H₂O solution | Contains PCR-grade water | LightCycler FastStart DNA Master HybProbe Kit, Roche Diagnostics | 1.9 |
| LightCycler Uracil-DNA Glycosylase | Enzyme solution containing Uracil-DNA Glycosylase | LightCycler Uracil-DNA Glycosylase, Roche Diagnostics | 0.5 |
| *Pseudomonas aeruginosa* Detection Mix | Primer and Hybridization Probe mixture specific for *Pseudomonas aeruginosa* serotype detection and internal control reaction | Kenta Biotech, present invention | 4 |
| **Volume of PCR premix solution per one PCR Reaction** | | | **10** |
| *Pseudomonas aeruginosa* Internal Control | Contains stabilized solution of internal control plasmid DNA. Solution is used as in-process control for template preparation or detection procedure. | Kenta Biotech, present invention | 2 |
| Template solution | Purified bacterial DNA, bacterial culture or processed clinical specimen. | e.g. clinical samples, etc. | 8 |
| As alternative to the combination of internal contol solution and template solution the positive control solution might be added to the PCR premix solution. | | | or |
| *Pseudomonas aeruginosa* Positive Control | Contains stabilized solution of positive control plasmid DNA. Solution is used as positive control in PCR amplification and result interpretation. | Kenta Biotech, present invention | 10 |
| **Final volume per one PCR reaction** | | | **20** |

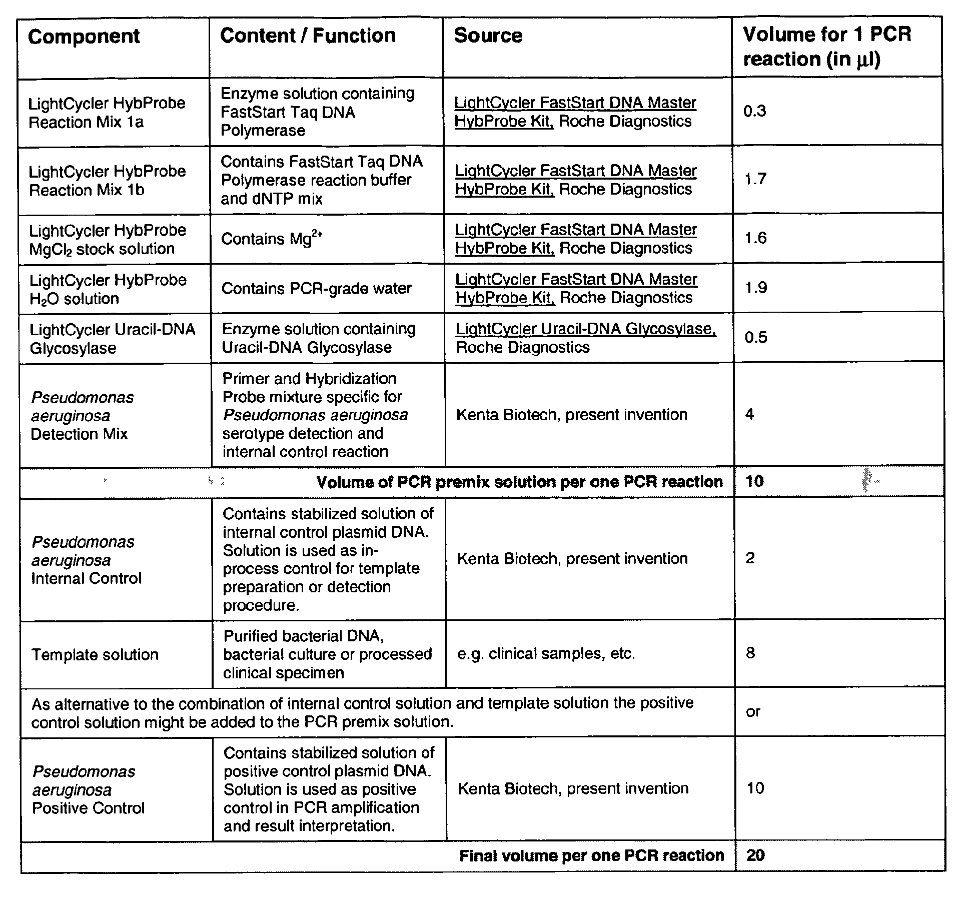

**Table 6**

| **4-valent *Pseudomonas aeruginosa* serotyping test - PCR positive control and internal control** For construction of both internal control and positive control plasmids, all amplicons detected by the 4-valent LightCycler assay were PCR-amplified from either reference strains or clinical isolates. After PCR amplification, resulting DNA fragments were purified and cloned into plasmid pT3T7BM (Roche Diagnostics) using the restriction sites *BamH*I/*Sac*I*.* Successful cloning of the control plasmids was finally verified by DNA sequencing. | | | | | |
|---|---|---|---|---|---|
| **Typ of Control** | **Plasmid** | **IATS Serotype** | **Reference Sequence** | **Reactive Primer/Probes** | **Concentration per PCR Reaction** |
| Positive | pPaer-O1-PC | O1 | NCBI accession AF498400; ORF_4; region 1284-2321 | O1 | 1.00E4 copies |
| | pPaer-S2-PC | S2 | NCBI accession AF498412; ORF_19; region 18769-19788 | S2 | 1.00E4 copies |
| | pPaer-O6-PC | 06 | NCBI accession AF498417; ORF_14; region 12654-13694 | O6 | 1.00E4 copies |
| | pPaer-O11-PC | O11 | NCBI accession AF498402; ORF_13; region 11073-12098 | O11 | 1.00E4 copies |
| Internal | pPaer-O1-IC | Based on 01 | NCBI accession AF498400; ORF_4; region 1284-2321 | O1 + IC | 20 copies |

### Example 5:

**The primers and probes according to the invention are suitable for specific detection of the clinically prevalent *P.aeruginosa* serotypes (IATS-01, serogroup 2 (IATS-02, IATS-O5, IATS-O16), IATS-06, and IATS-O11)**

**Table 7. Pseudomonas aeruginosa serotyping test**

| Six reference strains and 72 clinical isolates representing each of the 4 specified *Pseudomonas aeruginosa* serotypes IATS-O1, serogroup 2 (IATS-O2, IATS-O5, IATS-O16), IATS-O6, and IATS-O11 were detected by the 4-valent *Pseudomonas aeruginosa* serotyping test with 100% sensitivity and accuracy. Based on the design of the 4-valent *Pseudomonas aeruginosa* serotyping test, determination of strains belonging to either one of the detectable serotypes always resulted in one specific (positive) serotype detection signal, but three negative signals for the other serotype tests that are included in the multiplex- 4-valent- assay. For any negative serotyping test, the internal control was detected positive and as such any failure of the PCR method can be excluded. All 78 DNA samples were tested at approximately 10⁶ copies/reaction. | | | | |
|---|---|---|---|---|
| **IATS Serotype** | **Sample ID** | **Source** | **Sensitivity¹ [%]** | **Accuracy² [%]** |
| O11 | 1) LMG 14079 | BCCM, Reference strain ATCC 33358 | 100 | 100 |
| | 2) PEG4 | Clinical isolate Basel | 100 | 100 |
| | 3) PEG16 | Clinical isolate Basel | 100 | 100 |
| | 4) PEG19 | Clinical isolate Basel | 100 | 100 |
| | 5) PEG32 | Clinical isolate Basel | 100 | 100 |
| | 6) PEG35 | Clinical isolate Basel | 100 | 100 |
| | 7) PEG36 | Clinical isolate Basel | 100 | 100 |
| | 8) VA1014 | Clinical isolate Jena | 100 | 100 |
| | 9) VA26642/1 | Clinical isolate Jena | 100 | 100 |
| | 10) VA26794/4 | Clinical isolate Jena | 100 | 100 |
| | 11) VA26831 | Clinical isolate Jena | 100 | 100 |
| | 12) VA26939 | Clinical isolate Jena | 100 | 100 |
| | 13) VA2813 | Clinical isolate Jena | 100 | 100 |
| | 14) VA3805 | Clinical isolate Jena | 100 | 100 |
| | 15) VA3881 | Clinical isolate Jena | 100 | 100 |
| | 16) VA695 | Clinical isolate Jena | 100 | 100 |
| | 17) VA843 | Clinical isolate Jena | 100 | 100 |
| | 18) 2309.36 | Clinical isolate Berne | 100 | 100 |
| | 19) 2310.49 | Clinical isolate Berne | 100 | 100 |
| | 20) 2311.35 | Clinical isolate Berne | 100 | 100 |
| 06 | 21) CIP 59.39 | Institute Pasteur, Reference strain 33354 | 100 | 100 |
| | 22) PEG6 | Clinical isolate Basel | 100 | 100 |
| | 23) PEG20 | Clinical isolate Basel | 100 | 100 |
| | 24) PEG21 | Clinical isolate Basel | 100 | 100 |
| | 25) PEG22 | Clinical isolate Basel | 100 | 100 |
| | 26) PEG30 | Clinical isolate Basel | 100 | 100 |
| | 27)PEG33 | Clinical isolate Basel | 100 | 100 |
| | 28) 2311.11 | Clinical isolate Berne | 100 | 100 |
| | 29) 2310.15 | Clinical isolate Berne | 100 | 100 |
| | 30) 2312.18 | Clinical isolate Berne | 100 | 100 |
| | 31) 402/T512 | Clinical isolate Study 001 | 100 | 100 |
| | 32) 473/T323 | Clinical isolate Study 001 | 100 | 100 |
| | 33) 483/T406 | Clinical isolate Study 001 | 100 | 100 |
| | 34) 486/T429 | Clinical isolate Study 001 | 100 | 100 |
| | 35) 499/T338 | Clinical isolate Study 001 | 100 | 100 |
| | 36) 618/T348 | Clinical isolate Study 001 | 100 | 100 |
| 06 | 37) 599/T478 | Clinical isolate Study 001 | 100 | 100 |
| | 38) 575/T014 | Clinical isolate Study 001 | 100 | 100 |
| | 39) 581/T291 | Clinical isolate Study 001 | 100 | 100 |
| S2 - o2 | 40) LMG 14072 | BCCM, Reference strain o2 - ATCC 33356 | 100 | 100 |
| S2 - o5 | 41) LMG 14075 | BCCM, Reference strain o5 - ATCC 33352 | 100 | 100 |
| S2 - o16 | 42) LMG 14083 | BCCM, Reference strain o16 - ATCC 33363 | 100 | 100 |
| S2 - o5 | 43) PEG2 | Clinical isolate Basel | 100 | 100 |
| S2 - o5 | 44) PEG11 | Clinical isolate Basel | 100 | 100 |
| S2 - o16 | 45) PEG24 | Clinical isolate Basel | 100 | 100 |
| S2 - o16 | 46) PEG27 | Clinical isolate Basel | 100 | 100 |
| S2 - 05 | 47) PEG39 | Clinical isolate Basel | 100 | 100 |
| S2 - o2 | 48) PEG40 | Clinical isolate Basel | 100 | 100 |
| S2 - o2 | 49) UR538 | Clinical isolate Jena | 100 | 100 |
| S2 - o5 | 50) 2308.57 | Clinical isolate Berne | 100 | 100 |
| S2 - o16 | 51) 2310.27 | Clinical isolate Berne | 100 | 100 |
| S2 - o5 | 52) 2310.31 | Clinical isolate Berne | 100 | 100 |
| S2 - o5 | 53) 2312.58 | Clinical isolate Berne | 100 | 100 |
| S2 - o2 | 54) 348/T133 | Clinical isolate Study 001 | 100 | 100 |
| S2 - o2 | 55) 420/T358 | Clinical isolate Study 001 | 100 | 100 |
| S2 - o2 | 56) 421/T355 | Clinical isolate Study 001 | 100 | 100 |
| S2 - o2 | 57) 488/T183 | Clinical isolate Study 001 | 100 | 100 |
| S2 - o16 | 58) VA26925 | Clinical isolate Jena | 100 | 100 |
| S2 - o5 | 59) Vo56635 | Clinical isolate Zurich | 100 | 100 |
| O1 | 60) LMG 14071 | BCCM, Reference strain ATCC 33348 | 100 | 100 |
| | 61) PEG3 | Clinical isolate Basel | 100 | 100 |
| | 62) PEG7 | Clinical isolate Basel | 100 | 100 |
| | 63) PEG9 | Clinical isolate Basel | 100 | 100 |
| | 64) PEG12 | Clinical isolate Basel | 100 | 100 |
| | 65) PEG37 | Clinical isolate Basel | 100 | 100 |
| | 66) 2309.07 | Clinical isolate Berne | 100 | 100 |
| | 67) 2309.24 | Clinical isolate Berne | 100 | 100 |
| | 68) 2310.28 | Clinical isolate Berne | 100 | 100 |
| | 69) 2310.37 | Clinical isolate Berne | 100 | 100 |
| | 70) 2311.07 | Clinical isolate Berne | 100 | 100 |
| | 71) 2311.34 | Clinical isolate Berne | 100 | 100 |
| | 72) 2311.42 | Clinical isolate Berne | 100 | 100 |
| | 73) 417/T537 | Clinical isolate Study 001 | 100 | 100 |
| | 74) 448/T76 | Clinical isolate Study 001 | 100 | 100 |
| | 75) 471/T369 | Clinical isolate Study 001 | 100 | 100 |
| | 76) 485/T631 | Clinical isolate Study 001 | 100 | 100 |
| | 77) 487/T421 | Clinical isolate Study 001 | 100 | 100 |
| | 78) 615/T341 | Clinical isolate Study 001 | 100 | 100 |
| **Total Target Organisms** | | **78/78** | **100 %** | **100 %** |

| | | | | |
|---|---|---|---|---|
| ¹ : Sensitivity: number of detected isolates / number of isolates tested ²: Accuracy: number of correctly detected isolates / number of isolates detected | | | | |

### Example 6:

**The primers/probes of the invention do not show cross-reactivity with *P. aeruginosa* strains of other serotypes**

**Table 8: Pseudomonas aeruginosa serotyping test**

| The *Pseudomonas aeruginosa* serotyping test shows no cross-reactivity with reference strains and clinical isolates of the ten additional *Pseudomonas aeruginosa* serotypes IATS-O3, IATS-O4, IATS-O7, IATS-O8, IATS-O9, IATS-010, IATS-012, IATS-O13, IATS-O14, and IATS-015. All DNA samples were tested at approximately 10⁶ copies/reaction. In order to exclude the possibility that negative serotyping results are obtained because of potential PCR inhibitors that may be present in the sample, internal control solutions were added to each of the performed reactions. As the internal control was successfully detected from all 32 investigated serotype samples any PCR inhibition can be excluded. | | | | |
|---|---|---|---|---|
| **IATS Serotype** | **Sample ID** | **Source** | **Epidemiologic incidence** | # **Positive / # Tested [%]** |
| O3 | 1) LMG 14073 | BCCM, Reference strain ATCC 33350 | medium | 0 |
| | 2)2311.27 | Clinical isolate Berne | | |
| | 3) PEG23 | Clinical isolate Basel | | |
| | 4) PEG26 | Clinical isolate Basel | | |
| | 5) PEG34 | Clinical isolate Basel | | |
| O4 | 6) LMG 14074 | BCCM, Reference strain ATCC 33351 | medium | 0 |
| | 7) PEG38 | Clinical isolate Basel | | |
| | 8) 539/T179 | Clinical isolate Study 001 | | |
| | 9) 507/T642 | Clinical isolate Study 001 | | |
| 09 | 10) LMG 14077 | BCCM, Reference strain ATCC 33356 | medium | 0 |
| | 11)2309.06 | Clinical isolate Berne | | |
| | 12) 557/T535 | Clinical isolate Study 001 | | |
| | 13) 592/T450 | Clinical isolate Study 001 | | |
| | 14) Vo7 10013 | Clinical isolate Zurich | | |
| O10 | 15) LMG 14078 | BCCM, Reference strain ATCC 33357 | medium | 0 |
| | 16)2310.30 | Clinical isolate Berne | | |
| | 17) 567/T353 | Clinical isolate Study 001 | | |
| | 18) 559/T489 | Clinical isolate Study 001 | | |
| | 19) 598/T449 | Clinical isolate Study 001 | | |
| O7 | 20) CIP 59.38 | Institute Pasteur, Reference strain 33353 | rare | 0 |
| | 21) V10 8997 | Clinical isolate Zurich | | |
| | 22) 468/T599 | Clinical isolate Study 001 | | |
| O8 | 23) LMG 14076 | BCCM, Reference strain ATCC 33355 | rare | 0 |
| O8 | 24) 2310.02 | Clinical isolate Berne | rare | 0 |
| | 25) 2311.29 | Clinical isolate Berne | | |
| O12 | 26) LMG 14080 | BCCM, Reference strain ATCC 33359 | rare | 0 |
| | 27) UR 16217 | Clinical isolate Jena | | |
| O13 | 28) LMG 14081 | BCCM, Reference strain ATCC 33360 | rare | 0 |
| | 29) PEG14 | Clinical isolate Basel | | |
| O14 | 30) LMG 14429 | BCCM, Reference strain ATCC 33361 | rare | 0 |
| | 31) 16/T347 | Clinical isolate Study 001 | | |
| O15 | 32) LMG 14085 | BCCM, Reference strain ATCC 33362 | rare | 0 |
| **Total Target Organisms** | | **32/32** | **Unspecific detection: 0 %** | |

### Example 7:

**The primers according to the invention are species specific**

**Table 9: Pseudomonas aeruginosa serotyping test**

| Species specificity of the *Pseudomonas aeruginosa* serotyping test was proven by measurement of fifty eight bacterial, fungal, and viral microorganisms. Microbiological samples were obtained from American Type Culture Collection (ATCC), Deutsche Sammlung von Mikroorganismen und Zellkulturen (DSMZ) and Belgian Co-ordinated collections of Microorganisms (BCCM). All DNA samples evaluated showed no cross-reactivity with the 4-valent *Pseudomonas aeruginosa* serotyping test. All DNA samples were tested at approximately 10⁶ copies/reaction. In order to exclude the possibility that negative serotyping results are obtained because of potential PCR inhibitors that may be present in the sample, internal control solutions were added to each of the performed reactions. As the internal control was successfully detected from all 58 investigated species samples any PCR inhibition can be excluded. | | |
|---|---|---|
| **Microorganism** | **Species** | **# Positive/ # Tested [%]** |
| Bacteria Gram-negative | 1) Acinetobacter baumannii | 0 |
| | 2) Enterobacter aerogenes | |
| | 3) Enterobacter cloacae | |
| | 4) Escherichia coli | |
| | 5) Klebsiella oxytoca | |
| | 6) Klebsiella pneumoniae | |
| | 7) Proteus mirabilis | |
| | 8) Serratia marcescens | |
| | 9) Stenotrophomonas maltophilia | |
| | 10) Actinobacillus actinomycetemcomitans | |
| | 11) Aeromonas hydrophilia | |
| | 12) Bacterioides fragilis | |
| | 13) Bartonella henselae | |
| | 14) Bordetella pertussis | |
| | 15) Borrelia burgdorferi | |
| | 16) Burkholderia cepacia | |
| | 17) Campylobacter jejuni | |
| | 18) Cardiobacterium hominis | |
| | 19) Haemophilus influenza | |
| | 20) Legionella pneumophila | |
| | 21) Listeria monocytogenes | |
| | 22) Moraxella catarrhalis | |
| | 23) Morganella morganii | |
| | 24) Neisseria meningitidis | |
| | 25) Pantoea agglomerans | |
| | 26) Porphyromonas gingivalis | |
| | 27) Prevotella denticola | |
| | 28) Proteus vulgaris | |
| | 29) Yersinia enterocolitica | |
| Bacteria Gram-positive | 30) Enterococcus faecalis | 0 |
| | 31) Enterococcus faecium | |
| | 32) Staphylococcus aureus | |
| | 33) Staphylococcus epidermidis | |
| | 34) Staphylococcus haemolyticus | |
| | 35) Streptococcus pneumoniae | |
| Bacteria Gram-positive | 36) Streptococcus agalactiae | 0 |
| | 37) Streptococcus pyogenes | |
| | 38) Streptococcus mitis | |
| | 39) Bacillus cereus | |
| | 40) Clostridium perfringens | |
| | 41) Corynebacterium jeikeium | |
| | 42) Gemella haemolysans | |
| | 43) Histoplasma capsulatum | |
| | 44) Mycobacterium fortuitum | |
| | 45) Mycobacterium tuberculosis | |
| | 46) Mycoplasma pneumoniae | |
| | 47) Peptostreptococcus magnus | |
| | 48) Propionibacterium acnes | |
| Fungi | 49) Aspergillus fumigatus | 0 |
| | 50) Candida albicans | |
| | 51 Candida glabrata | |
| | 52) Candida krusei | |
| | 53) Candida parapsilosis | |
| | 54) Candida tropicalis | |
| | 55) Cryptococcus neoformans | |
| Viruses | 56) Herpes simplex virus, type 1 (HHV-1/HSV-1) | 0 |
| | 57) Herpes simplex virus, type 2 (HHV-2/HSV-2) | |
| Viruses | 58) Cytomegalovirus, type 1 (HHV-5/CMV) | 0 |
| **Total Target Organisms** | | **Unspecific detection: 0%** |

### Example 8:

### Reproducibility / Robustness of the method

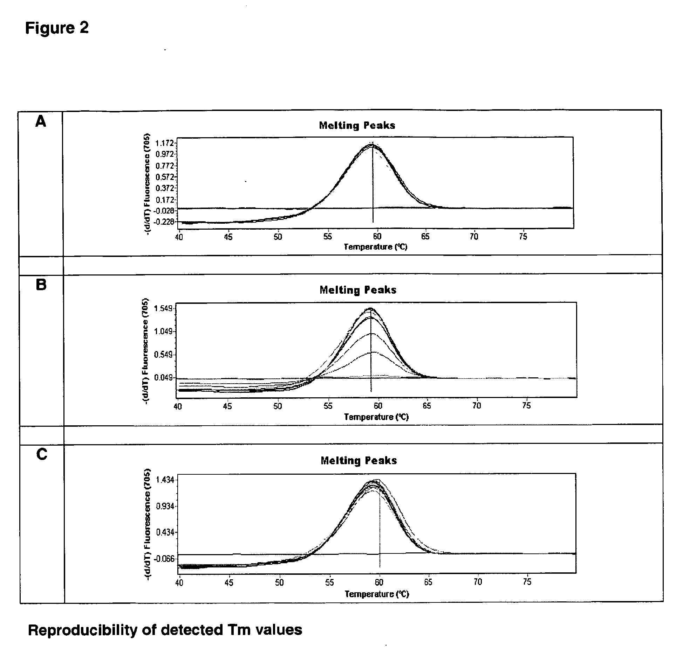

High reproducibility of serotype detection in different experimental setups is shown for serotype IATS-O6 exemplarily. Identical Tm values are obtained as shown in Figure 2.
(A) from measurement of reference strain and 19 clinical isolates,
(B) independent of the applied template concentrations (10² to 10⁷ genomes/reaction)
(C) or after measurement of 30 clinical samples (broncho-alveolar lavage)

### Example 9:

### Analytical Sensitivity

The analytical sensitivity of the *Pseudomonas aeruginosa* serotyping test was determined by hit-rate analysis of *Pseudomonas aeruginosa* IATS-O1, serogroup 2 (IATS-02, IATS-O5 and IATS O-16), IATS-O6, and IATS-O11 reference strains using purified DNA in 8-fold replicates of seven target-positive samples. For each microbial DNA tested the 95% cut-off value was calculated to determine the lower limit of detection (LOD) using Probit analysis. The multiplex format of the 4-valent serotyping test might influence the LOD of each of the four tests. It can be assumed that each of the four assays has a lower LOD when tested separately.

**Table 10:**

| **4-valent *Pseudomonas aeruginosa* serotyping test - Analytical sensitivity** | | | | | | | | |
|---|---|---|---|---|---|---|---|---|
| **IATS O1 Reference Strain, ATCC 33348** | | | | | | | | |
| **Concentration (gene copies/PCR)** | 300 | 150 | 75 | 50 | 12.5 | 3.125 | 1.56 | 0 |
| **Hit rate** | 8/8 | 8/8 | 7/8 | 6/8 | 4/8 | 0/8 | 0/8 | 0/8 |
| **Detection Limit, Calculated LOD95 value** | 101.55 | | | | | | | |
| | | | | | | | | |

| **IATS S2 (02) Reference Strain, ATCC 33356** | | | | | | | | |
|---|---|---|---|---|---|---|---|---|
| **Concentration (gene copies/PCR)** | 300 | 150 | 75 | 50 | 12.5 | 3.125 | 1.56 | 0 |
| **Hit rate** | 8/8 | 8/8 | 8/8 | 6/8 | 3/8 | 0/8 | 1/8 | 0/8 |
| **Detection Limit, Calculated LOD95 value** | 101.53 | | | | | | | |
| | | | | | | | | |

| **IATS 06 Reference Strain, ATCC 33354** | | | | | | | | |
|---|---|---|---|---|---|---|---|---|
| **Concentration (gene copies/PCR)** | 300 | 150 | 75 | 50 | 12.5 | 3.125 | 1.56 | 0 |
| **Hit rate** | 8/8 | 8/8 | 8/8 | 8/8 | 3/8 | 2/8 | 1/8 | 0/8 |
| **Detection Limit, Calculated LOD95 value** | 56.16 | | | | | | | |
| | | | | | | | | |

| **IATS O11 Reference Strain, ATCC 33358** | | | | | | | | |
|---|---|---|---|---|---|---|---|---|
| **Concentration (gene copies/PCR)** | 300 | 150 | 75 | 50 | 12.5 | 3.125 | 1.56 | 0 |
| **Hit rate** | 8/8 | 8/ 8 | 7/8 | 5/8 | 2/8 | 0/8 | 0/8 | 0/8 |
| **Detection Limit, Calculated LOD95 value** | 121.36 | | | | | | | |

### Example 10:

### Comparison: Serotyping of clinical isolates by 4-valent Pseudomonas aeruginosa serotyping test and slide agglutination.

Respiratory *Pseudomonas aeruginosa* isolates were collected at approximately twenty hospitals in the United States, Germany, Greece, and Belgium. Measurements were performed after oN incubation of the bacterial strains on BHI agar plates (BD Bioscience) at 37°C. One inoculation loop of each of the strains was used for agglutination as well as for dilution in ultrapure H₂O followed by subsequent LightCycler analysis.
When comparing the results obtained from both serotyping methods, it is obvious that the number of reliably identified clinical isolates can be dramatically increased by application of the 4-valent *Pseudomonas aeruginosa* serotyping test. In absolute numbers the observed improvement corresponds to fifteen percent of the five hundred investigated bacterial strains (improvement of serotype determination from 54.3 % to 69.2 %). In this particular example the difference of fifteen percent corresponds to seventy-five potential patients that would have become eligible for serotype-specific antibody treatment.

**Table 11:**

| **IATS Serotyp** | **Place of Isolate Collection** | | | | **Sum** |
|---|---|---|---|---|---|
| | **US** | **Germany** | **Greece** | **Belgium** | |
| Number of Isolates tested | 94 | 186 | 100 | 119 | 499 |
| **Serotype Distribution [%]** | | | | | **Average [%]** |
| **Serotyping by Slide Agglutination** | | | | | |
| O1 | 106 | 8.6 | 2.0 | 6.7 | 7 |
| S2 | 6.4 | 4.3 | 9.0 | 6.7 | 6.6 |
| O6 | 19 1 | 20.4 | 8.0 | 19.3 | 16.7 |
| O11 | 20.2 | 16.7 | 38.0 | 21.0 | 24 |
| **Sum of Serotypes O1, S2, O6, and O11 that were detected by Slide Agglutination** | **56.3** | **50.0** | **57.0** | **53.7** | **54.3** |
| Non-typeable Isolates | 25.5 | 23.7 | 26 | 23 5 | 24.7 |
| | | | | | |
| **Serotyping by 4-valent PCR assay** | | | | | |
| O1 | 14.9 | 13.4 | 4.0 | 7.6 | 10.0 |
| S2 | 7.4 | 7.5 | 12.0 | 11.8 | 9.7 |
| O6 | 25.5 | 24.2 | 11.0 | 24.4 | 21.3 |
| O11 | 25 5 | 17.2 | 30.0 | 30 3 | 25.8 |
| **Sum of Serotypes 01, S2, 06, and O11 that were detected by 4-valent PCR Serotyping Test** | **73.3** | **62.3** | **67.0** | **74.1** | **69.2** |
| Serotypes different from 01, S2, 06, and O11 | 26.7 | 37.7 | 33.0 | 25,9 | 30.8 |

### Example 11:

### 4-valent Pseudomonas aeruginosa serotyping test - non-functional Primer/Probe pairs.

For serotype specific detection of *Pseudomonas aeruginosa* isolates specific primer/probe oligonucleotides are of need. For the design of such sequences the LightCycler Probe Design Software 2.0 (Roche Applied Science) was used. Although the software proposed many oligonucleotide sequences that fulfill the general requirements of good primer and probe design (e.g. no intra-molecular sequence homologies, single annealing sites on template, amplicon size, desired melting temperature, etc.), many of the proposed primer/probe pairs could not be used for reliable serotype identification because of the enumerated exclusion criteria.

**Table 12:**

| | | | | | |
|---|---|---|---|---|---|
| Twenty five (25) primer/probe oligonucleotides that were designed using the LightCycler Probe Design Software 2.0 (Roche Applied Science) were depicted. None of the primer/probe pairs could be used to generate reliable serotype specific detection signals over a broad range of template concentrations. | | | | | |

| **Name Primer / Probe Pair** | **IATS Serotype Detection** | | **Target Gen** | **Primer / Probe Sequence 5' - 3'** | **Exclusion criteria** |
|---|---|---|---|---|---|
| Wzz_O1_hk1 O1_wzz_P1/2 | O1 | | Wzz, similar to chain length determinant protein; NCBI accession AF498400; ORF_4; region 1284-2321 | for: GTGGCGGTGAAATTGATCTC | Undesired variation of the serotype specific detection signal and low signal intensity |
| | | | | rev: GCAACACGGCGATACTG | |
| | | | | P1: TTATCCTGGTCGTAGCTCTTATATTTGG CATTG | |
| | | | | P2: GCTGCTATATATGCCTATACAAGTAAG CCGGTT | |
| Wzz_O1_hk7 O1_wzz_P3/4 | | | | for: CCTGGTCGTAGCTCTTATATT rev: CTGAAGACCCGACTCCT P3: GCCAGTATCGCCGTGTTGC P4:CCCTCGCTGAGCAATGTTGCCG | Undesired variation of the serotype specific detection signal and low signal intensity |
| Wzz_O1_hk10 O1_wzz_P5/6 | | | | for: ATCAGTATTACTCTGCCAGACA rev: GTGCGCATTCTTAACCATTTC P5:TGAGCTGGCCGCTGAGTG P6:TCCGTCGCTATCTTGCCGATACGG | Undesired variation of the serotype specific detection signal |
| Wzz_O1_hk11 O1_wzz_P7/8 | | | | for: CGGTGAAATTGATCTCGTAAGAC | Undesired variation of the serotype specific detection signal and low signal intensity |
| | | | | rev: GGCAACATTGCTCAGCG | |
| | | | | P7: TAGCTCTTATATTTGGCATTGTTGCTGC TA | |
| | | | | P8: TATGCCTATACAAGTAAGCCGGTTTAT GAAGCCAG | |
| Wzz_O1_hk4 O1_wzz_P9/10 | | | | for: CGCGATAGTCTCTATAGGGTATTTA rev: CCATTTCTTGGACGGAATGT P9: ACAGTAGAACAGGAAGATCCTGAGC P10: GCCGCTGAGTGGATCCGTCG | Undesired variation of the serotype specific detection signal |
| 6 | Wzz_S2_hk4 S2_wzz_P3/4 | S2 | Wzz, similar to chain length determinant protein; NCBI accession AF498412; region 1288-2334 | P3: TTTCAATGAGACCTATTTGCCTTCTTTG GA | Undesired variation of the serotype specific detection signal |
| | | | | for: CTTCAAGGTCCAGGATGTG | |
| | | | | rev: GGCAAACTAATACTTATCTGATCAGTG | |
| | | | | P4:AAGAGCTTCGTTCGGTTTCGCG | |
| | | | | | |
| 7 | Wzz_S2_hk6 S2_wzz_P7/8 | | | P8: GGGTTCGTCGGTATATAGCTGATGCG G | Undesired variation of the serotype specific detection signal |
| | | | | for: ACTGATCAGATAAGTATTAGTTTGCC | |
| | | | | rev: GCATTGTTCAACATTTCCTGAATAGA | |
| | | | | P7:GGAGCGTGCGGCGAG | |
| 8 | GtGr4_S2_hk9 S2_GtGr4_P5/6 | | GtGr4, similar to glycosyltransfera se group 4 NCBI accession ACTTCATGGATGGCATTGATGGTATTG AF498412; ORF_19; region 18769-19788 | for: CTGCTCGGCCATTTCTC rev: ACACTGGCAATACCATCAA P5: GTTTCCCGCCGCTGGAT P6: GGTTGGGCATGCTGTCGACTTAGG | Insufficient sensitivity |
| 9 | GtGr4_S2_hk18 S2_GtGr4_P7/8 | | | for: GGCATGCTGTCGACTTAG rev: CATGCCCTGTAAGCCAGTA P7: ACTTCATGGATGGCATTGATGGTATTG P8:AGTGTCGAGGCCATTGGTGTCTG | Undesired variation of the serotype specific detection signal |
| 10 | GtGr4_S2_hk24 S2_GtGr4_P9/10 | | | P9: TATCCCTCTGTTGCTGGCGTGCCCATG AAGATT | Insufficient sensitivity |
| | | | | for: CAGTGTCGAGGCCATTG rev: CGCATCA P10: CGGTCGCCGGCTTCCTGA | |
| 11 | Ored_02_hk1 | S2 | Similar to Oxidoreductase family, NAD binding Rossmann fold NCBI accession AF498412; ORF_6; region 4525-5475 | for:TAAGCGCCTCTACAACATTCT rev: TGGATCTCCCTTCCAGC | No serotype specificity |
| 12 | Ored_O2_hk3 | | | for:AGCGTAATGTTGTGCACTTCA rev: ACGGTAATCGAACGATAGG | No serotype specificity |
| 13 | Ored_O2_hk4 | | | for: GTCCGCATAAGTACGAGG rev: GCAGCTTGCCAAAGATGA | No serotype specificity |
| 14 | Asp_O6_hk1 | O6 | Similar to Aspargine synthase NCBI accession AF498417; ORF_10; region 7493-9376 | for: TCCGATGTTCCTTTGGGAG rev: CAACCGCCTTTGCGTAA | No serotype specificity |
| 15 | Asp_O6_hk2 | | | for: GCAGTTGATCTTCTGGAAAGT rev: CAACCGCCTTTGCGTAA | No serotype specificity |
| 16 | Asp_O6_hk3 | | | for: CCGAGGCAGTTGATCTTC rev: CAGGCTCATCAAAGCCAAT | No serotype specificity |
| 17 | Asp_O6_hk4 | | | for: AGCATATCGGATCAGATGG rev: CGTAAACAGCCTCATCATACC | No serotype specificity |
| 18 | Asp_O6_hk5 | | | for:TCCGATGTTCCTTTGGGAG rev: GTACCGATATGTTCTGCAACC | No serotype specificity |
| 19 | GtGr4_O11_hk41 O11_GtGr4_P1/2 | O11 | GtGr4, similar to glycosyltransfera se group 4 NCBI accession | for:ATGGATGGAATCGATGGAATTG rev: GCAGGAGGGAAATTCCAGA P1: TGTGTTGGCGGGGCATTATTATACTG P2:TGAATGGCCAACTGACGCAGGC | Undesired variation of the serotype specific detection signal |
| GtGr4_O11_hk1 O11_GtGr4_P3/4 | | AF498402; ORF_13; region 11073-12098 | | for: TAGTGCAGCCTTGGTCT rev: CGATCCCATCCATGAAGTTAT P3:TGTTGGTGTCAGTTGGGACCTG P4: GTGGTTCGGAGGACTTCTCTTTGCTTT | Low signal intensity |
| GtGr4_O11_hk3 O11_GtGr4_P9/10 | | | | P9: TGTGGTTGCTGAATCTCTATAACTTCAT GG | Insufficient sensitivity |
| | | | | CCATTTCAGATTGTTGGTGTCA ATGCCCTAATTCAGCCAGTATAA P10:GGGATCGATGGACTTGCCAGCC | |
| GtGr4_O11_hk4 O11_GtGr4_P11/12 | | | | P11: TCTATCTCGTGTGGTTGCTGAATCTCT A | Insufficient sensitivity |
| | | | | for: GCAGCCTTGGTCTCATTGTA rev:ATGCCCTAATTCAGCCAGTATAA P12: ACTTCATGGATGGGATCGATGGACTTG | |
| Wzz_O11_hk1 O11_wzz_P1/2 | | Wzz, similar to chain length determinant protein NCBI accession AF498402; ORF_4 region 919-1956 | | P2: AAGATAGATGGCCCGCCATTAATAGAA GGG | Undesired variation of the serotype specific detection signal |
| | | | | for: TGCAGAGCCGCATAACC rev: TCCATGATCGAGGAAAGTTGT P1: GCTGATTGCGGAGTCGC | |
| Wzz_O11_hk16 O11_wzz_P3/4 | | | | for:TCCTGCTAACAAGCCAGATG rev: CTGGAAATCTCTACCTGCACTA P3: GCGAGAGGTTCTTGCTACATGG P4: ACAAGCTTTCGTGCGTTTGGCTG | Undesired variation of the serotype specific detection signal |
| Wzz_O11_hk30 O11_wzz_P7/8 | | | | for: GAGCGGAAAGCGAAGATGA rev: AAAGCTTGTGCCCATGT P7:TAACAAGCCAGATGCAGACCG P8: ATACGGTAATTGTGGAGGGCACGAAG | Undesired variation of the serotype specific detection signal |

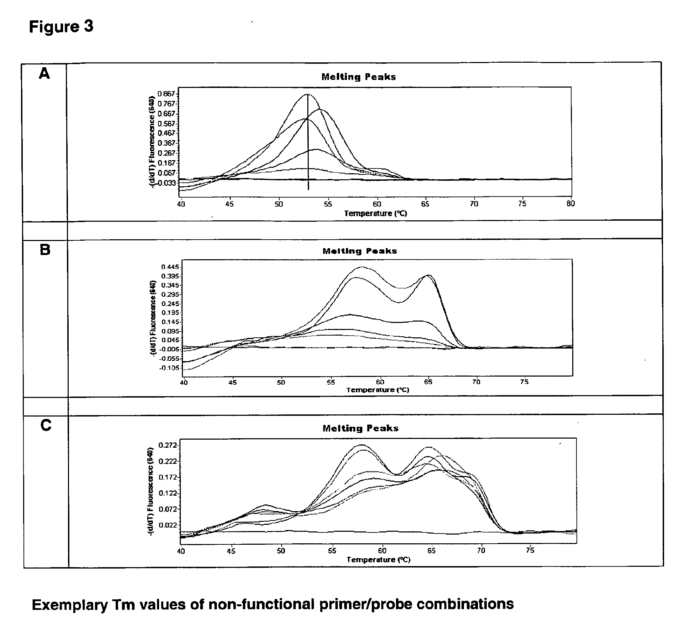

Figure 3 shows three non functional primer/probe combinations which were generated by using the LightCycler Probe Design Software.

In case of strong deviation of an amplification-specific Tm value the corresponding primer/probe pairs can not be used for reliable serotype identification. Figure 3 shows three examples with strong Tm variation in dependence of the applied template concentration. In all three experiments 10e6 to 10e2 genomes/reaction were measured using the reference strains of IATS-O11 (A), IATS-02 (B), and IATS-O1 (C). The samples depicted are corresponding to the primer/probe combinations #19 (A), #9 (B), and #1 (C) of table 12.

### References:

Chenna R, Sugawara H, Koike T, Lopez R, Gibson TJ, Higgins DG, Thompson JD. (2003). Multiple sequence alignment with the Clustal series of programs. Nucleic Acids Res., 31, 3497-3500.
Connolly BA. (1987). The synthesis of oligonucleotides containing a primary amino group at the 5'-terminus. Nucleic Acids Res., 10, 3131-3139.
Gait MJ, Sigh M, Sheppard RC (1980) Rapid synthesis of oligodeoxyribonucleotides IV. Improved solid phase synthesis of oligodeoxyribonucleotides through phosphotriester intermediates Nucl. Acid Res 8:1081
Raymond CK, Sims EH, Kas A, Spencer DH, Kutyavin TV, Ivey RG, Zhou Y, Kaul R, Clendenning JB, Olson MV. (2002). Genetic variation at the O-anticien biosynthetic locus in Pseudomonas aeruginosa. J Bacteriol.;184, 3614-3622.
Rivera, M., T.R. Chivers, J.S. Lam, and E.J. McGroarty. (1992). Common antigen lipopolysaccharide from Pseudomonns aeruginosa Auk 1401 as a receptor for bacteriophage A7. J. Bacteriol. 174:2407-2411.

## Claims

1. An assay for serotype-specific detection of *Pseudomonas aeruginosa* in a sample comprising the steps of:
a) annealing at least one pair of *Pseudomonas aeruginosa* serotype-specific primers to a target nucleic acid in a sample, wherein the at least one pair of *Pseudomonas aeruginosa* serotype-specific primers is selected from the group consisting of:
i) a first oligonucleotide primer comprising at least 10 consecutive nucleotides of the sequence shown in SEQ ID No 1 and a second oligonucleotide primer comprising at least 10 consecutive nucleotides of the sequence shown in SEQ ID No 2,
ii) a first oligonucleotide primer comprising at least 10 consecutive nucleotides of the sequence shown in SEQ ID No 3 and a second oligonucleotide primer comprising at least 10 consecutive nucleotides of the sequence shown in SEQ ID No 4,
iii) a first oligonucleotide primer comprising at least 10 consecutive nucleotides of the sequence shown in SEQ ID No 5 and a second oligonucleotide primer comprising at least 10 consecutive nucleotides of the sequence shown in SEQ ID No 6, and
iv) a first oligonucleotide primer comprising at least 10 consecutive nucleotides of the sequence shown in SEQ ID No 7 and a second oligonucleotide primer comprising at least 10 consecutive nucleotides of the sequence shown in SEQ ID No 8;
b) amplifying the target nucleic acid, and
c) detecting the *Pseudomonas* amplified target nucleic acid.

2. The assay according to claim 1, wherein the step of detecting comprises the step of hybridizing at least one pair of *Pseudomonas aeruginosa* serotype-specific hybridization probes to a sequence internal to the sequences where the at least one *Pseudomonas aeruginosa* serotype-specific primer pair selected in claim 1 anneals.

3. The assay according to claim 2, wherein the at least one pair of *Pseudomonas aeruginosa* serotype-specific hybridization probes is selected from the group consisting of:
(i) a first oligonucleotide probe comprising at least 10 consecutive nucleotides of the sequence shown in SEQ ID No 9 and a second oligonucleotide probe comprising at least 10 consecutive nucleotides of the sequence shown in SEQ ID No 10,
(ii) a first oligonucleotide probe comprising at least 10 consecutive nucleotides of the sequence shown in SEQ ID No 11 and a second oligonucleotide probe comprising at least 10 consecutive nucleotides of the sequence shown in SEQ ID No 12,
(iii) a first oligonucleotide probe comprising at least 10 consecutive nucleotides of the sequence shown in SEQ ID No 13 and a second oligonucleotide probe comprising at least 10 consecutive nucleotides of the sequence shown in SEQ ID No 14, and
(iv) a first oligonucleotide probe comprising at least 10 consecutive nucleotides of the sequence shown in SEQ ID No 15 and a second oligonucleotide probe comprising at least 10 consecutive nucleotides of the sequence shown in SEQ ID No 16.

4. The assay according to claims 2 or 3, wherein the *Pseudomonas aeruginosa* serotype-specific hybridization probe is labeled with a detectable marker.

5. The assay according to claim 4, wherein the detectable marker is selected from the group consisting of: luminescent markers, fluorescent markers, radioactive markers and enzyme markers.

6. The assay according to any one of claims 2 to 5, wherein the *Pseudomonas aeruginosa* serotype is detected by quantification, amplification curve or melting curve analysis.

7. The assay according to any one of claims 1 to 6, wherein two or more the *Pseudomonas aeruginosa* serotypes are detected simultanously.

8. The assay according to claim 7, wherein the serotypes to be detected are *Pseudomonas aeruginosa* serotypes IATS-01, IATS-06, IATS-011 and serogroup 2, wherein serogroup 2 contains serotypes IATS-02, IATS-05 and IATS-016.

9. The assay according to any one of claims 1 to 8, wherein the sample is selected from the group of bodily materials such as sputum, broncho-alveolar lavage, tracheal aspiration, blood, urine, tissue, and DNA isolated from bacterial culture.

10. The assay according to any one of claims 1 to 8, wherein the sample is selected from the group of: food, soil and water.

11. The assay according to any one of claims 1 to 10, wherein the bacterial loads in the sample are detectable in the range of 10 cfu/ml to 10⁹ cfu/ml.

12. The assay according to any one of claims 1 to 11, wherein the assay is species-specific.

13. An oligonucleotide comprising a nucleotide sequence selected from the sequence shown in SEQ ID Nos.: 1-16.

14. A kit for the serotype-specific detection of *Pseudomonas aeruginosa* comprising at least one *Pseudomonas aeruginosa* serotype-specific primer pair selected from the group consisting of:
a. a first oligonucleotide primer comprising at least 10 consecutive nucleotides of the sequence shown in SEQ ID No 1 and a second oligonucleotide primer comprising at least 10 consecutive nucleotides of the sequence shown in SEQ ID No 2,
b. a first oligonucleotide primer comprising at least 10 consecutive nucleotides of the sequence shown in SEQ ID No 3 and a second oligonucleotide primer comprising at least 10 consecutive nucleotides of the sequence shown in SEQ ID No 4,
c. a first oligonucleotide primer comprising at least 10 consecutive nucleotides of the sequence shown in SEQ ID No 5 and a second oligonucleotide primer comprising at least 10 consecutive nucleotides of the sequence shown in SEQ ID No 6, and
d. a first oligonucleotide primer comprising at least 10 consecutive nucleotides of the sequence shown in SEQ ID No 7 and a second oligonucleotide primer comprising at least 10 consecutive nucleotides of the sequence shown in SEQ ID No 8; and reagents required for annealing, and optionally reagents for amplification and instructions for the serotype-specific detection of *Pseudomonas aeruginosa.*

15. The kit according to claim 14, wherein the kit further comprises at least one pair of *Pseudomonas aeruginosa* serotype-specific hybridization probes that hybridizes to a sequence internal to the sequences where the at least one serotype-specific primer pair selected in claim 14 anneals.

16. The kit according to claim 15, wherein the at least one pair of *Pseudomonas aeruginosa* serotype-specific hybridization probes are selected from the group consisting of:
a. a first oligonucleotide probe comprising at least 10 consecutive nucleotides of the sequence shown in SEQ ID No 9 and a second oligonucleotide probe comprising at least 10 consecutive nucleotides of the sequence shown in SEQ ID No 10,
b. a first oligonucleotide probe comprising at least 10 consecutive nucleotides of the sequence shown in SEQ ID No 11 and a second oligonucleotide probe comprising at least 10 consecutive nucleotides of the sequence shown in SEQ ID No 12,
c. a first oligonucleotide probe comprising at least 10 consecutive nucleotides of the sequence shown in SEQ ID No 13 and a second oligonucleotide probe comprising at least 10 consecutive nucleotides of the sequence shown in SEQ ID No 14, and
d. a first oligonucleotide probe comprising at least 10 consecutive nucleotides of the sequence shown in SEQ ID No 15 and a second oligonucleotide probe comprising at least 10 consecutive nucleotides of the sequence shown in SEQ ID No 16.

17. The kit according to claims 15 or 16, wherein the *Pseudomonas aeruginosa* serotype-specific probe is labeled with a detectable marker.

18. The kit according to claim 17, wherein the detectable marker is selected from the group consisting of: luminescent markers, fluorescent markers, radioactive markers and enzyme markers.

19. The kit according to any one of claims 14 to 18, wherein the *Pseudomonas aeruginosa* serotypes IATS-01, IATS-06, IATS-011 and serogroup 2, wherein serogroup 2 contains serotypes IATS-02, IATS-05 and IATS-016, are detected simultaneously.

20. Use of an antibody specific for one or more of *Pseudomonas aeruginosa* serotype IATS-01, IATS-06, IATS-011 and serogroup 2, wherein serogroup 2 contains serotypes IATS-02, IATS-05 and IATS-016, for the preparation of a medicament for the treatment of a *Pseudomonas aeruginosa* infection in a patient, in whom said specific *Pseudomonas aeruginosa* serotype according to the assay of claim 1-12 has been detected.

21. An antibody specific for one or more of the *Pseudomonas aeruginosa* serotypes IATS-01, IATS-06, IATS-011 and serogroup 2, wherein serogroup 2 contains serotypes IATS-02, IATS-05 and IATS-016 for the use in treating *Pseudomonas aeruginosa* infection in a patient, in whom said specific *Pseudomonas aeruginosa* serotype according to the assay of claim 1-12 has been detected.

22. Use of the oligonucleotide primers or probes as defined in claim 1 or 3 for serotype-specific detection of *Pseudomonas aeruginosa.*


SEQUENCE LISTING

<110>  Kenta Biotech AG

<120>  Primer for Detection of Pseudomonas aeruginosa Serotypes

<130>  EP66870HV200pau

<160>  21

<170>  PatentIn version 3.3

<210>  1
<211>  21
<212>  DNA
<213>  Artificial

<220>
<223>  oligonucleotide primer

<400>  1
tacgccgtga agatagaatt g                                                21

<210>  2
<211>  18
<212>  DNA
<213>  Artificial

<220>
<223>  oligonucleotide primer

<400>  2
tctcctctaa gcgccttg                                                    18

<210>  3
<211>  17
<212>  DNA
<213>  Artificial

<220>
<223>  oligonucleotide primer

<400>  3
ttcatgggtg atgcggg                                                     17

<210>  4
<211>  17
<212>  DNA
<213>  Artificial

<220>
<223>  oligonucleotide primer

<400>  4
tccggcggat cagagta                                                     17

<210>  5
<211>  19
<212>  DNA
<213>  Artificial

<220>
<223>  oligonucleotide primer

<400>  5
tgtactggct tggaggttt                                                    19


<210>  6
<211>  20
<212>  DNA
<213>  Artificial

<220>
<223>  oligonucleotide primer

<400>  6
atcggaacct acaccactga                                                   20


<210>  7
<211>  17
<212>  DNA
<213>  Artificial

<220>
<223>  oligonucleotide primer

<400>  7
tagtgcagcc ttggtct                                                      17


<210>  8
<211>  21
<212>  DNA
<213>  Artificial

<220>
<223>  oligonucleotide primer

<400>  8
cgatcccatc catgaagtta t                                                 21


<210>  9
<211>  20
<212>  DNA
<213>  Artificial

<220>
<223>  oligonucleotide primer

<400>  9
tgggcaggta tctgagcagc                                                   20


<210>  10
<211>  34
<212>  DNA
<213>  Artificial

<220>
<223>  oligonucleotide primer

<400>  10
tcggctatca tgaacggtag cttgatgtat atgc                                   34


<210>  11
<211>  15
<212>  DNA
<213>  Artificial

<220>
<223>  oligonucleotide primer

<400>  11
ccctcgctgt tctgg                                                        15


<210>  12
<211>  35
<212>  DNA
<213>  Artificial

<220>
<223>  oligonucleotide primer

<400>  12
gttgatattg ctgggagtgt tcatcgttga tgcaa                                  35


<210>  13
<211>  16
<212>  DNA
<213>  Artificial

<220>
<223>  oligonucleotide primer

<400>  13
cagcgttgag gctgtg                                                       16


<210>  14
<211>  16
<212>  DNA
<213>  Artificial

<220>
<223>  oligonucleotide primer

<400>  14
tgtgtgcgtt ggcgct                                                       16


<210>  15
<211>  21
<212>  DNA
<213>  Artificial

<220>
<223>  oligonucleotide primer

<400>  15
ttggtgtcac ttgggacctg g                                                 21


<210>  16
<211>  27
<212>  DNA
<213>  Artificial

<220>
<223>  oligonucleotide primer

<400>  16
tggttcggag gacttctctt tgctttc                                           27

<210>  17
<211>  34
<212>  DNA
<213>  Artificial

<220>
<223>  oligonucleotide primer

<400>  17
agccgatagt acttgccatc gaactacata tacg                                34


<210>  18
<211>  1038
<212>  DNA
<213>  Pseudomonas aeruginosa

<400>  18
atggtagcgt tgaaagaaga aaaaaacggt ggcggtgaaa ttgatctcgt aagacttttt      60

cagattctat ggaagggaaa gtggattatc ctggtcgtag ctcttatatt tggcattgtt     120

gctgctatat atgcctatac aagtaagccg gtttatgaag ccagtatcgc cgtgttgcca     180

ccctcgctga gcaatgttgc cggttttaac caggggcgta ccaaggagtc gggtcttcag     240

gcatttaaag ttcaggatgt ctattccgtg ttcttgcgca atcttcaagc tgatgagact     300

cggcaccgct tcttcgagaa tgtctacttg ccgtcgctag gaggtgagag gccggcgtcg     360

cgcgatagtc tctatagggt atttaatgaa aagatcagta ttactctgcc agacaagacc     420

cagccgagtc gctatcggct aacagtagaa caggaagatc ctgagctggc cgctgagtgg     480

atccgtcgct atcttgccga tacggccgaa cattccgtcc aagaaatggt taagaatgcg     540

caccgtgaga tagaggtcaa ggctcgggat gtcgatcagc ggatccaaac gctacgtgaa     600

actgctaagt tacgccgtga agatagaatt gtgcaactta gagaggctct aaaagtcgcg     660

gatgctgtgg ggttggaaaa gcctccgatg ataagtgggc aggtatctga gcagctttcg     720

gctatcatga acggtagctt gatgtatatg cggggcagca aggcgcttag aggagagatc     780

gaggctttgg aaagtcgtgt gtcagatgat cctttcatcc ctgctttacg aactctgcag     840

gagcaggaaa aactctatag tagtttacgg gtaaacaccg acgaggttgc tgtgttccgt     900

caggatggtt cggttgagac gcccgattct cccgtgaagc cgaagaagct tttactcgtc     960

atgatggggt tgttgctcgg tggtgcggtt ggtgtcgtga ctgtcgtgtt taattcgctc    1020

tatcgcaata agcggtaa                                                  1038


<210>  19
<211>  1020
<212>  DNA
<213>  Pseudomonas aeruginosa

<400>  19
atgatgatct ggatgatcgc gtgtctagtt gtcttgctgt tttcatttgt cgctacctgg      60

gggctgcgtc gctatgcatt agcgacgaaa ctgatggatg ttccgaatgc ccgtagctcc     120

cacagtcaac cgacgcctag ggggggaggt gttgcaatcg ttctggtctt ccttgcagcg     180

ttggtgtgga tgctgagtgc aggcagtatc tccggcggct gggggggggc gatgctgggt     240

gcaggttctg gcgtggcact gttagggttc ctggatgacc atgggcacat tgctgcgcgt 300
tggcggctgc tcggccattt ctcagcagcg atatggatct tgctgtggac gggtggtttc 360
ccgccgctgg atgtggttgg gcatgctgtc gacttaggat ggctgggcca cgtattggca 420
gttttctatt tggtatgggt gctgaacctt tataacttca tggatggcat tgatggtatt 480
gccagtgtcg aggccattgg tgtctgtgta ggaggggccc tgatctactg gcttacaggg 540
catgtcgcga tggttggtat ccctctgttg ctggcgtgcg cggtcgccgg cttcctgatc 600
tggaacttcc ctccagctcg aatcttcatg ggtgatgcgg ggagtggttt tcttggtatg 660
gttattggtg cactagctat tcaggctgca tggaccgccc cctcgctgtt ctggtgctgg 720
ttgatattgc tgggagtgtt catcgttgat gcaacctata ctctgatccg ccggatagcc 780
agaggggaga aattctatga ggcgcatcgc agccacgctt atcagtttgc ctcgcgccgt 840
tatgctagcc atctgcgggt taccttgggt gttctggcta tcaacactct ttggttgttg 900
ccgttggcac tgatggttgc attgggttgg atcagcggct tcatcggtat cctggttgct 960
tatgctcctc tttgcctctt ggcggtagga tacaaggcgg gttccttgga aaaatcctaa 1020

<210> 20
<211> 1041
<212> DNA
<213> Pseudomonas aeruginosa

<400> 20
atgatgaatc tatggttgtt gttgccggcg gttgccgctc tttccctgct gttgaccgcc 60
ggcttgcgcc gttacgcgat tgcccgcagc ttgatcgatg tacccaatgc tcgaagttct 120
caccaggtgc cgacgcctag ggggggcggt gtcgctatcg tgctctcctt cctgctggca 180
gtgctgcttg cggcgatatt gggcgcggtg aaaccggatc tggccactgg tatcctgggg 240
gcgggtattg ggatcgcatt gctgggcttc ctcgacgatc atggccatat cgctgcccgt 300
tggcgtttgc tcggccattt cgccggtgct tgctggctgc tgtactggct tggaggtttg 360
cctgcattgg cctttttcgg ccttgtggtc gatctggggt gggttggaca catagctgcg 420
gcgttctatc tggtatggat gctcaatctc tacaatttca tggacggtat cgatgggatt 480
gccagcgttg aggctgtgtg tgtgtgcgtt ggcgctgccc tgcttgtcgt ggtcagtggt 540
gtaggttccg atgaggcatc ccagggcgtt tggttggcgg cgctgctggc agccgcggtg 600
acggggttcc ttttctggaa tttccctcct gcgcggatct tcatggggga tgctggtagc 660
ggtttcttgg gggttatcat tggtggcctt tccctgcaag ccgcatgggt ctcgccgcag 720
ctcttctggg gttggctcat cctgctgggg gtattcattg ttgatgccac cctgaccttg 780
ttgcgccggt tgctgcgggg agacaaggtt tatgaggcgc atcgcagtca cgcataccag 840
tatgcttctc gtcattacgg gcgtcatctt cctgttaccc tggcagtggg aggcatcaac 900
atcttctggt tgttgccact ggcactgctt gtcgcagcgg ggaagatcga cggcatgctt 960
gctttgttga ttggctacct gcctctggcg ttcctggcgc tccgttttaa ggccggagtc 1020

ctggaatcac gcgctgcttg a                                              1041

<210>  21
<211>  1026
<212>  DNA
<213>  Pseudomonas aeruginosa

<400>  21
atggaagaat ggtatttgtt actcgctgca gctggggttt cgggactgct tacaggcctc      60

ttgcgtcgtt atgccttagc gaggagctta cttgacaccc ctaactctcg aagttcccat     120

gtcgttccca ctccacgcgg aggaggggtc gccattgtag ttactttttg tctcatgctg     180

cctatttggg ctgtactggg aaatatctca tgggccgtgt cctgggcttt acttctcgct     240

ggcggcgggg ttgccattat tggattcatg gatgatcacg gtcatatcgc cgcacgctgg    300

cgtctgctgg gacattttag tgcagccttg gtctcattgt actttttgaa tggcatacca     360

ccatttcaga ttgttggtgt cagttgggac ctggggtggt tcggaggact tctctttgct     420

ttctatctcg tgtggttgct gaatctctat aacttcatgg atgggatcga tggacttgcc     480

agccttcagg ccatttttgt ctgtgttggc ggggcattat tatactggct gaatggccaa     540

ctgacgcagg ctttgctccc cttatcgcta gcttttgccg tttttggatt cttgttctgg     600

aattttccac ccgcaaaaat tttcatggga gatgcgggta gtggttttct ggggattgtt     660

ttaggaattc tttccattca tgccatgtgg atgaatacga attttttctg ggcatggttg      720

gtcctgttag gcgttttcat cgtcgatgcg acctataccc tgattcgtcg cttgctgaga     780

ggggacaagg tgtatgaggc tcatcgaagc catgcctatc aatacgcaag ccgatactat     840

ggaaagcatg ctcctgttac gattggcgtc acggcattga acgtcatctg gctcctccct     900

atagccttgt tggtcgggag tggatctcta gagcctttga tgggcatcgt catagcctac     960

gtccctctcg tttttctggc agtgaggttc aaggcgggta agctagagtc gtccgctcag    1020

gcctaa                                                               1026